# EUROPÄISCHE PATENTSCHRIFT

(11) **EP 1 465 602 B1**
(45) Veröffentlichungstag und Bekanntmachung des Hinweises auf die Patenterteilung: **05.08.2009**
(21) Anmeldenummer: 02796612.6
(22) Anmeldetag: 12.12.2002
(51) Int. Cl.: A61K 9/16, A61K 9/28, A61K 9/20

(54) **MATRIX-FILMTABLETTE MIT KONTROLLIERTER FREISETZUNG VON NATÜRLICHEN GEMISCHEN KONJUGIERTER OESTROGENE**
MATRIX FILM TABLETS FOR THE CONTROLLED RELEASE OF NATURAL MIXTURES OF CONJUGATED ESTROGEN
COMPRIME PELLICULE MATRICIEL A LIBERATION CONTROLEE DE MELANGES NATURELS D'OESTROGENES CONJUGUES

(30) Priorität: 14.12.2001 EP 01129830
(43) Veröffentlichungstag der Anmeldung: 13.10.2004
(73) Patentinhaber: Solvay Pharmaceuticals GmbH, 30173 Hannover (DE)
(72) Erfinder: THUMBECK, Bernd, 31171 Nordstemmen (DE); BUDDE, Klaus, 31535 Neustadt (DE); KRISTEN, Gerhard, 31303 Burgdorf (DE); WIARDS, Margit, 30890 Barsinghausen (DE)
(74) Vertreter: Gosmann, Martin
(86) Internationale Anmeldenummer: PCT/EP2002/014103
(87) Internationale Veröffentlichungsnummer: WO 2003/051336

(56) Entgegenhaltungen:
- EP-A- 0 322 020
- WO-A-97/04752
- US-A- 5 908 638

## Beschreibung

Die vorliegende Erfindung betrifft eine pharmazeutische Matrix-Filmtablette mit kontrollierter Freisetzung von natürlichen Gemischen konjugierter Oestrogene, die aus dem Harn trächtiger Stuten gewonnenen wurden.

Oestrogene werden in der Medizin zur Hormonsubstitutionstherapie eingesetzt. Insbesondere werden Oestrogengemische zur Behandlung und Prophylaxe der bei Frauen auftretenden Beschwerden der Wechseljahre nach natürlicher oder artifizieller Menopause eingesetzt. Hierbei haben sich natürliche Gemische konjugierter Oestrogene, wie sie im Harn trächtiger Stuten vorliegen, als besonders wirksam und gut verträglich erwiesen.

Der gelöste Feststoffgehalt im Harn trächtiger Stuten (= pregnant mares' urine, im folgenden abgekürzt als "PMU") kann natürlicherweise in weiten Bereichen schwanken und im allgemeinen in einem Bereich von 40 bis 90 g Trockensubstanz pro Liter liegen. Neben Harnstoff und sonstigen üblichen Harninhaltsstoffen sind im Feststoffgehalt des PMU phenolische Bestandteile, z.B. Kresole und das als HPMF bekannte Di-hydro-3,4-bis[(3-hydroxyphenyl)methyl]-2(3H)-furanon, enthalten. Das im PMU enthaltene natürliche Gemisch von Oestrogenen liegt weitgehend in konjugierter Form, z.B. als Schwefelsäurehalbester-Natriumsalz (im folgenden abgekürzt als "Sulfatsalz"), vor. Der Gehalt an konjugierten Oestrogenen (= conjugated estrogen, im folgenden abgekürzt als "CE") kann berechnet als Oestrogensulfatsalz und bezogen auf Trockensubstanz zwischen 0,3 und 1 Gew.-% betragen.

Bei der Abtrennung der unerwünschten Begleitstoffe, wie Harnstoff und insbesondere Kresole und HPMF, werden aus dem PMU üblicherweise Extrakte gewonnen, welche die konjugierten Oestrogene aus dem Harn trächtiger Stuten (PMU) in gelöster Form enthalten. Neuere Methoden gewinnen hierbei natürliche Gemische dieser konjugierten Oestrogene (CE) durch Festphasenextraktion des Gemisches konjugierter Oestrogene aus dem Harn trächtiger Stuten z.B an RP-Kieselgel (WO 98/08525) oder an nichtionischen semipolaren polymeren Adsorberharzen (WO 98/08526). Zwar können die unerwünschten Begleitstoffe mit diesen Methoden effektiver und effizienter Weise aus dem PMU abgetrennt und wäßrige Extrakte der CE von guter Qualität gewonnen werden, doch unterliegt die Konzentration der CE im Extrakt gewissen, unvermeidlichen Schwankungen, da PMU als ein natürliches Ausgangsmaterial für die Gewinnung der CE per se natürlichen Qualitätsschwankungen aufgrund Herkunft, Lagerung, Transport und eventueller Vorverarbeitung unterliegt.

Aufgrund der Eigenschaften der derart gewonnenen Extrakte von natürlichen Gemischen konjugierter Oestrogene und insbesondere auch aufgrund der üblicherweise darin nach Aufarbeitung noch verbliebenen Begleitstoffe, ist es nicht einfach, diese Extrakte galenisch in feste pharmazeutische Zubereitungen mit verläßlicher Qualität zu überführen. Bei der Herstellung von festen pharmazeutischen Zubereitungen von natürlichen Gemischen konjugierter Oestrogene aus CE-enthaltenden Extrakten muß jedoch eine gleichbleibende Qualität und Dosierungsstärke der Zubereitung sowie ein vorgegebenes Freisetzungsprofil sichergestellt werden. Die in Abhängigkeit von Ausbeute und Qualität des PMU-Ausgangsmaterials auftretenden natürlichen Schwankungen des Gehaltes konjugierter Oestrogene in den für die Herstellung pharmazeutischer Zubereitungen verwendeten Extrakten müssen daher durch geeignete galenische Verarbeitung ausgeglichen werden, damit feste pharmazeutische Zubereitungen von natürlichen Gemischen konjugierter Oestrogene mit gleichbleibender Qualität, Dosierungsstärke sowie vorgegebenem Freisetzungsprofil bereitgestellt werden können.

Aufgabe der vorliegenden Erfindung ist somit die Bereitstellung verbesserter fester pharmazeutischer Zubereitungen von natürlichen Gemischen konjugierter Oestrogene, welche die gestellten pharmazeutischen Anforderungen erfüllen und in einfacher Weise in gleichbleibender Qualität, Dosierungsstärke sowie mit vorgegebenem Freisetzungsprofil hergestellt werden können. Insbesondere ist es Aufgabe der Erfindung feste pharmazeutische Zubereitungen von natürlichen Gemischen konjugierter Oestrogene, die aus dem Harn trächtiger Stuten gewonnen wurden, bereitzustellen, die das natürliche Gemisch konjugierter Oestrogene als Wirkkomponente in definierter Form und Konzentration sowie in homogener Verteilung in einer durch Tablettierung, beispielsweise Direkttablettierung, von Wirkstoff-haltigen pulver- oder partikelförmigen Rohstoffen hergestellten Matrix-Filmtablette enthalten und eine vorgegebenes Freisetzungsprofil aufweisen.

In der internationalen Anmeldung WO 97/04752 wird ein Verfahren zur Herstellung einer konjugierte Östrogene enthaltenden Matrixtablette mit kontrollierter Wirkstofffreisetzung beschrieben, in dem konjugierte Östrogene in definierter Ausgangsmenge in einem hydroalkoholischen Lösungsmittel gelöst und dann auf organischer Trägerstoffe aufgesprüht werden, wobei diese Trägerstoffe eine Kombination aus gelbildenden, das Freisetzungsverhalten beeinflussenden organischen Polymeren wie CelluloseDerivate, beispielsweise Hydroxypropylmethylcellulose, und nicht gelbildenden organischen Trägerstoffen wie Laktose sind. Nach Aufsprühen einer definierten Menge an konjugierten Östrogenen auf die Mischung der im Wirbelschichtgerät fluidisierten organischen Trägerstoffe wird unter Zugabe weiterer Tablettierhilfsstoffe granuliert, anschließend unter definierten Bedingungen getrocknet, dann das so erhaltene Granulat mit geeigneten Bindemitteln vermischt und zu Tabletten verpresst.

Die Europäische Anmeldung EP 0 322 020 beschreibt die Bereitstellung pharmazeutischer Kombinationspräparate enthaltend Desogestrel (ein Progesteron) und mindestens ein Alkalimetallsalz eines konjugierten Ostrogens aus der Gruppe umfassend Estron, Equilin, 17 alpha Dihydroequilin, 17 alpha Estradiol, Equilenin, 17 beta Dihydroequilin und 17 beta Dihydroequilenin. Beispielhaft wird die Herstellung von Tabletten beschrieben, wobei zunächst die pharmazeutischen Trägerstoffe wie Laktose, Stärke, mikrokristalline Cellulose und Hydroxypropylmethylcellulose (HPMC) gemischt, und die Mischung dann in einem Granulierungsprozess unter Zugabe einer alkoholischen Lösung von alpha-Tocopherol und 5-Descogestrol und anschließend unter Zugabe einer wässrigen Lösung, welche die konjugierten Östrogene in definiertem Verhältnis (nämlich Natrium Estronsulfat, Natrium Equilinsulfat und Natrium 17 alpha Dihydroequilinsulfat im Gewichtsverhältnis 6:3:1) sowie Tromethamin und Natriumcitrat enthält, granuliert, abschließend das Granulat getrocknet, gemahlen und mit Stearinsäure, Magnesiumstearat und Siliziumdioxide verschmiert und in Tabletten verpresst wird.

Es wurde nun überraschenderweise gefunden, daß pulver- oder granulatförmige Trockenextrakte bzw. Präformulierungen von natürlichen, aus PMU gewonnen Gemischen konjugierter Oestrogene (CE), die durch Aufsprühen eines wäßrigen CE-enthaltenden Lösungsextrakte auf einen, in einer Wirbelschicht fluidisierten festen pharmazeutischen Trägerstoff in homogener Verteilung und definierter Konzentration hergestellt wurden, Trockenextrakte bzw. Präformulierungen von hoher Qualität darstellen, die galenisch durch Tablettierung, beispielsweise Direkttablettierung, gewünschtenfalls auch nach vorhergehender Granulierung, bequem zu Matrixtabletten mit definierter und homogener Wirkstoffverteilung weiterverarbeitet werden können.

Die vorliegende Erfindung betrifft daher eine pharmazeutische Matrix-Filmtablette mit kontrollierter Wirkstofffreisetzung, wobei sich die Tablette durch folgende Merkmale auszeichnet:
(a) einen wasserquellbaren Matrixkern,
   (i) der als Wirkstoff ein natürliches Gemisch konjugierter equiner Oestrogene in Form eines pharmazeutisch präformulierten homogenen Trockenextraktes eingebettet enthält, wobei der Trockenextrakt einen pro Menge Trägermaterial definierten, (bezogen auf die Haupthormon-Komponenten) standardisierten Wirkstoffgehalt des Gemisches natürlicher konjugierter equiner Oestrogene enthält, und der Wirkstoffgehalt durch Aufsprühen aus einer wäßrigen Lösung auf einen pulver- und/oder granulatförmigen pharmazeutischen Trägerstoff aus der Gruppe mikrokristalliner Cellulosen oder einem Gemisch von mikrokristalliner Cellulose mit Lactose und Trocknung aufgebracht ist;
   (ii) dessen wasserquellbare Matrix aus einer tablettierbaren Zusammensetzung gebildet wird, die wenigstens einen Gerüstbildner (Matrixbildner) aus der Gruppe gelbildender pharmazeutischer Polymere, insbesondere ein gelbildendes pharmazeutisches Polymer aus der Gruppe der Cellulose- und/oder Stärkederivate, sowie gegebenenfalls einen oder mehrere weitere pharmazeutische Tablettierhilfsstoffe aus der Gruppe anderer Gerüstbildner, wie insbesondere mikrokristalline Cellulosen, der Füllstoffe, der Bindemittel, der wasserlöslichen osmotischen Agenzien und der Gleitmittel, wie insbesondere Fließregulierungsmittel, Schmiermittel, und/oder Formtrennmittel, umfaßt
   und daß die Tablette
(b) mit einem den Matrixkern umgebenden, filmbildenden Überzug versehen ist, dessen Zusammensetzung
   (i) wenigstens einen hydrophoben pharmazeutische Filmbildner und weiterhin
   (ii) gegebenenfalls pharmazeutische Weichmacher und/oder Porenbildner und/oder ein hydrophiles Polymer umfaßt.

Aus den erfindungsgemäßen Matrix-Filmtabletten kann der Wirkstoff, z.B. das Estron-Sulfat und/oder auch andere Hormonkomponenten, über 8 bis 12 Stunden kontrolliert mit einer starken Verzögerung in den ersten 2 Stunden freigesetzt werden, wobei die Freisetzungskinetik gegebenenfalls durch ein in der Matrix enthaltenes osmotisches Agenz weiter kontrolliert werden kann.

Als Matrix-Filmtabletten im Rahmen der vorliegenden Erfindung werden Matrixtabletten verstanden, die einen mit einer Filmbeschichtung umhüllten Matrixkern aufweisen. Matrixtabletten sind sogenannte Gerüsttabletten, die den Wirkstoff in einem Polymer- oder Wachsgerüst bzw. -matrix eingebettet enthalten. Die in Matrixtabletten verwendeten Hilfsstoffe, wie z.B. Polymere, lösen sich in wäßrigen Medien nicht sofort auf bzw. erodieren nicht sofort, so daß die in die Matrix eingebundenen Wirkstoffe nicht sofort, sondern im Laufe einer verzögerten Erosion bzw. langsamen Ablösung des polymeren matrixbildenden Hilfsstoffes freigesetzt werden. Tabletten und Matrixtabletten sowie deren Herstellung sind dem galenischen Fachmann geläufig (siehe z.B. K. H. Bauer et al., Pharmazeutische Technologie, 1986 Thieme-Verlag, S. 374-390). Matrixtabletten bzw. Matrixkernen werden üblicherweise wie Tabletten generell in Tablettiervorichtungen gepreßt. Matrixtabletten oder Matrixkerne enthalten neben dem für die verzögerte Freisetzung verantwortlichen Matrixbildner regelmäßig weitere übliche pharmazeutische Tablettierhilfsstoffe. Solche Hilfsstoffe, die üblicherweise zum Tablettieren verwendet werden, umfassen z.B. Füllstoffe, Bindemittel oder Klebestoffe, z.B. bei vorheriger Feuchtgranulierung, Trockenbindemittel bei vorheriger Trockengranulierung und bei Direkttablettierung, ggf. Feuchthaltemittel, Trocken- oder Adsorptionsmittel, Gleitmittel wie Fließregulierungs-, Schmier- oder Formtrennmittel, und ggf. weitere Hilfmittel mit anderen oder zusätzlichen Funktionen. Polymere, die eine verzögerte Freisetzung des Wirkstoffes bedingen können, sind auch als Trockenbindemittel einsetzbar. Neben der normalen Tablettierung, bei der regelmäßig Bindemittel z.B. für eine vorhergehende Granulierung, eingesetzt werden, ist in der pharmazeutischen Technologie auch die Direkttablettierung gebräuchlich, z.B. wenn selbstbindende Trägerstoffe eingesetzt werden. Bei der Direkttablettierung wird auf einen Granulierschritt verzichtet und die einzelnen Träger- und Hilfsstoffe werden direkt mit dem Wirkstoff verpreßt.

Die im Rahmen der vorliegenden Erfindung geeigneten Trägerstoffe müssen daher einige Grundvoraussetzungen erfüllen, beispielsweise müssen sie eine Matrix für die Einbettung des Wirkstoffes bilden und sich zur Tablettierung, ggf. zur Direkttablettierung eignen.

Im Rahmen der vorliegenden Erfindung kommen vielfältige gelbildende pharmazeutische polymere Trägerstoffe, die sich zur Tablettierung, beispielsweise Direkttablettierung, eignen, in Betracht. Im Rahmen der vorliegenden Erfindung verwendete Träger- und Hilfsstoffe für den Tablettenkern sind z.B. mikrokristalline Cellulose, Lactose, Natriumchlorid, Magnesiumstearat, ggf. Calciumtriphosphat, Polymere wie z.B. Hydroxypropylcellulose, und wenn ein Granulierschritt zwischengeschaltet wird, Hydroxypropylmethylcellulosen. In zweckmäßigen Ausgestaltungen der vorliegenden Erfindung zeichnet sich die Matrix-Filmtablette durch die Auswahl von gelbildenden pharmazeutischen polymeren Trägerstoffen aus der Gruppe der Cellulosederivate, vorzugsweise aus der Gruppe Hydroxypropylcellulose (HPC), Hydroxypropylmethylcellulose (HPMC) und/oder Carboxymethylcellulose (CMC) aus.

Im Rahmen der vorliegenden Erfindung kommen weiterhin zusätzlich zum gelbildenden pharmazeutischen polymeren Trägerstoff vielfältige weitere übliche galenische Hilfsstoffe für die Tablettenherstellung, insbesondere weitere Hilfstoffe für die Tablettierung, beispielsweise Direkttablettierung, in Betracht. Solche Hilfsstoffe können z.B. zur gezielten Variation der Tabletteneigenschaften - z.B. Wirkstoff-Freisetzungscharakteristik, Tablettenfestigkeit oder Modifizierung der Verarbeitbarkeit der zu pressenden Mischung - in der Matrixtablette enthalten sein. Die zusätzlichen Hilfsstoffe können daher z.B. weitere Trägerstoffe - auch nicht-matrixbildende Trägerstoffe, wasserlösliches osmotisches Agens oder Schmiermittel sein.

In einer vorteilhaften Ausgestaltung der erfindungsgemäßen Matrix-Filmtablette weist der Matrixkern wenigstens einen gelbildenden pharmazeutischen polymeren Trägerstoff aus der Gruppe der Cellulosederivate und als weitere Hilfsstoffe wenigstens eine mikrokristalline Cellulose als zusätzlichen Trägerstoff und Lactose als wasserlösliches Agens und Natriumchlorid als osmotisches Agens auf. Die Mengenmäßige Zusammensetzung bevorzugter Matrixkerne dieser Variante der Erfindung ist dadurch charakterisiert, daß die wasserquellbare Matrix 20 bis 50 Gew.-Teile eines gelbildenden pharmazeutischen polymeren Trägerstoffes aus der Gruppe der Cellulosederivate und als weitere pharmazeutische Hilfsstoffe 10 bis 30 Gew.-Teile einer mikrokristallinen Cellulose und 40 bis 70 Gew.-Teile eines wasserlöslichen osmotischen Agens enthält. Bevorzugt sind hierbei solche erfindungsgemäßen Matrix-Filmtabletten, in denen die wasserquellbare Matrix 20 bis 50 Gew.-Teile Hydroxypropylcellulose (HPC) als gelbildenden pharmazeutischen polymeren Trägerstoff und als weitere pharmazeutische Hilfsstoffe 10 bis 30 Gew.-Teile mikrokristalline Cellulose und 40 bis 70 Gew.-Teile Lactose als wasserlösliches Agens und 0,1 bis 3 Gew.-% Natriumchlorid als osmotisches Agens enthält.

Im Rahmen der vorliegenden Erfindung kann es in bestimmten Varianten zweckmäßig sein, wenn die Matrix-Filmtablette zusätzlich zu den Matrix-bildenden Trägerstoffen und ggf. sonstigen Hilfsstoffen im Matrixkern als weiteren Hilfsstoff ein Schmiermittel enthält. Für die Tablettenherstellung geeignete Schmiermittel sind dem Fachmann geläufig. Als Beispiele seien Stearate genannt. Vorzugsweise wird im Rahmen der vorliegenden Erfindung Magnesiumstearat als Schmiermittel verwendet. Das Schmiermittel kann in der erfindungsgemäßen Matrix-Filmtablette in einer Menge im Matrixkern enthalten sein, die bezogen auf 100 Gew.-Teile der wasserquellbaren Matrix 0,1 bis 5 Gew.-Teile, vorzugsweise 2 bis 5 Gew.-Teile entspricht.

Als Wirkstoffkomponente ist in der erfindungsgemäßen Ma trix-Filmtablette ein natürliches Gemisch konjugierter Oestrogene, die aus dem Harn trächtiger Stuten gewonnenen wurden, enthalten. Solche natürlichen Gemische konjugierter Oestrogene enthalten üblicherweise eine ganze Reihe von Hormonen, die in unterschiedlichen Konzentrationen vorliegen. Als Hormone können in diesen natürlichen Gemischen üblicherweise 17-α-Estradiol, 17-β-Estradiol, 17-α-DH-Equilin, 17-β-DH-Equilin, 17-α-DH-Equilenin, 17-β-DH-Equilenin, Estron, Equilin, δ-8,9-Dehydroestron und Equilenin enthalten sein. Die wesentlichen Hormonkomponenten sind hierbei 17-α-Estradiol, 17-α-DH-Equilin, 17-β-DH-Equilin, Estron und Equilin, wobei insbesondere Estron und Equilin mengenmäßig die beiden Hauptbestandteile des natürlichen Gemisches der konjugierten Oestrogene bilden. Die konjugierten Oestrogene liegen in der erfindungsgemäßen Matrix-Filmtablette vorzugsweise in einem auf die gesamte Matrix-Filmtablette (d.h. einschließlich der weiter unten beschriebenen Filmüberzuges) als 100 Gew.-% bezogenen und als Trockensubstanz des Gemisches natürlicher konjugierter Oestrogene berechneten Wirkstoffgehalt vor, der im für pharmazeutische Zubereitungen mit konjugierten equinen Oestrogenen üblichen Bereich liegt. Die erfindungsgemäße Matrix-Filmtablette kann alternativ oder ergänzend zum vorstehenden Trockensubstanzgehalt auch durch ihren Gesamthormongehalt charakterisiert werden.

In erfindungsgemäß vorteilhafter Weise sind die genannten natürlichen Gemische der konjugierten Oestrogene nicht direkt, z.B. nicht direkt aus einem CE-enthaltenden Lösungsextrakt, in den wasserquellbaren polymeren matrixbildenden Trägerstoff eingearbeitet, sondern in Form eines präformulierten homogenen Trockenextraktes auf einem pharmazeutischen Trägerstoff. Das natürliche Gemisch der konjugierten Oestrogene liegt somit auf einem separaten Trägerstoff als Trockenextrakt bzw. feste Präformulierung vor, über die es quasi indirekt in der Matrix homogen eingebettet ist. Zweckmäßige Ausgestaltungen der erfindungsgemäßen Matrix-Filmtablette zeichnen sich daher durch ihren auf den Matrixkern bezogenen Gehalt an wirkstoffhaltiger Präformulierung aus, wobei dieser Gehalt, insbesondere unter Berücksichtigung des Wirkstoffgehaltes in der Präformulierung, in weiten Breichen variieren kann. Der hierbei im Matrixkern als Wirkstoffkomponente enthaltene homogene Trockenextrakt ist insbesondere ein, durch Aufsprühen aus einer wäßrigen Lösung auf einen ursprünglich pulver- oder granulatförmigen pharmazeutischen Trägerstoff aus der Gruppe mikrokristalline Cellulose oder einem Gemisch von mikrokristalliner Cellulose mit Lactose und Trocknung, auf dem pharmazeutischen Trägerstoff in homogener Verteilung präformuliertes natürliches Gemisch konjugierter Oestrogene. Weitere Details über die Wirkstoff-haltigen Präformulierungen sind weiter unten im Zusammenhang mit dem erfindungsgemäßen Verfahren zur Herstellung der Matrix-Filmtabletten angegeben.

Die erfindungsgemäßen Matrix-Filmtabletten sind weiterhin dadurch charakterisiert, daß sie einen Filmüberzug besitzen, also einem den Matrixkern umgebenden Überzug aufweisen. Erfindungsgemäß wird der Überzug aus einer Filmzusammensetzung gebildet wird, die zwingend wenigstens einen hydrophoben pharmazeutischen Filmbildner und fakultativ weiterhin pharmazeutische Weichmacher und/oder Porenbildner umfaßt.

Zweckmäßige hydrophobe pharmazeutische Filmbildner im Rahmen der vorliegenden Erfindung sind insbesondere solche, die schlecht wasserlöslich sind und die eine Ablösung des Films durch Zugabe von hydrophilen Zuschlagstoffen oder Porenbildnern zulassen. Beispiele solcher hydrophoben pharmazeutischen Filmbildner sind Polymethacrylate oder Polymetha crylat-Derivate. Bevorzugter hydrophober pharmazeutischer Filmbildner im Rahmen der vorliegenden Erfindung ist Polymethacrylat.

Zweckmäßige Weichmacher im Rahmen der vorliegenden Erfindung sind insbesondere solche, die die Plastizität des Filmes verbessern und eine Hydrophilisierung des Filmes bewirken oder als Porenbildner fungieren. Ein Beispiel und bevorzugter Weichmacher im Rahmen der vorliegenden Erfindung ist Triethylcitrat.

Zweckmäßige Porenbildner im Rahmen der vorliegenden Erfindung sind insbesondere solche, die durch Wasser oder wäßrige Medien leicht angelöst oder aufgelöst werden und durch dieses Herauslösen aus dem Film Poren erzeugen. Beispiele solcher Porenbildner sind Polyethylenglykole vom Typ 6000 (PEG 6000) oder Porenbildner vom Typ Hydroxypropymethylcellulose (HPMC). Diese Porenbildner sind im Rahmen der vorliegenden Erfindung bevorzugt.

In einer bevorzugten Ausgestaltung der erfindungsgemäßen Matrix-Filmtabletten zeichnen sich diese dadurch aus, daß der Überzug als hydrophoben pharmazeutischen Filmbildner ein Polymethacrylat, als Weichmacher z.B. Triethylcitrat und als Porenbildner Polyethylenglykol 6000 (PEG 6000) und/oder HPMC, sowie gegebenenfalls Talkum umfaßt. In dieser Variante enthält der Überzug bezogen auf den Matrixkern als 100 Gew.-% beispielsweise das Polymethacrylat in einer Menge von 0,1 bis 1 Gew.-%, das Triethylcitrat in einer Menge von 0,05 bis 0,5 Gew.-% und Polyethylenglykol 6000 in einer Menge von 0,01 bis 0,5 Gew.-% und/oder Hydroxypropylmethylcellulose in einer Menge von 0,01 bis 0,5 Gew.-%.

Erfindungsgemäß können die Matrix-Filmtabletten in verschiedenen Tablettenstärken vorliegen. Zweckmäßig sind insbesondere Matrix-Filmtabletten, die einschließlich Überzug eine Tablettenstärke mit einem Gesamtgewicht von 0,3 mg, 0,625 mg, 0,9 mg, 1,25 mg oder 2,5 mg aufweisen. Die erfindungsgemäßen Matrix-Filmtabletten können dabei je nach Tablettenstärke unterschiedliche Freisetzungsprofile für das als Wirkstoff enthaltene natürliche Gemisch konjugierter Oestrogene aufweisen. In einer Variante der Erfindung zeigt die erfindungsgemäße Matrix-Filmtablette für die Tablettenstärken von 0,3 mg und 0,625 mg ein Freisetzungsprofil mit einer als Summe von Estron und Equilin gemessenen Wirkstofffreisetzung von 19 bis 49 % in 2 Stunden, 66 bis 96 % in 5 Stunden und > 80 % nach 8 Stunden. In einer weiteren Variante der Erfindung zeigt die erfindungsgemäße Matrix-Filmtablette für die Tablettenstärken von 0,9 mg und 0,625 mg ein Freisetzungsprofil mit einer als Summe von Estron und Equilin gemessenen Wirkstofffreisetzung von 12 bis 37 % in 2 Stunden, 57 bis 85 % in 5 Stunden und > 80 % nach 8 Stunden. In einer anderen Variante der Erfindung zeigt die erfindungsgemäße Matrix-Filmtablette für die Tablettenstärken von 1,25 mg und 2,5 mg ein Freisetzungsprofil mit einer als Summe von Estron und Equilin gemessenen Wirkstofffreisetzung von 3 bis 22 % in 2 Stunden, 37 bis 67 % in 5 Stunden, 6 bis 96 % in 8 Stunden und > 80 % nach 12 Stunden.

Weiterhin können die erfindungsgemäßen Matrix-Filmtabletten gewünschtenfalls zusätzlich eine an sich übliche Zuckerdragierung aufweisen. Die Zuckerdragierung dient dem Zweck des Abdichtens der Tablette gegen Luft und Feuchtigkeit. Die Zuckerdragierung kann in einer an sich üblichen Menge auf den Matrix-Filmtabletten aufgebracht sein, d.h. in einer Menge, die ausreicht den vorstehenden Zweck zu erfüllen.

Die vorliegende Erfindung betrifft auch ein Verfahren zur Herstellung der oben beschriebenen erfindungsgemäßen Matrix-Filmtablette. Dieses Verfahren zur Herstellung der beschriebenen erfindungsgemäßen Matrix-Filmtablette ist **dadurch gekennzeichnet, daß** man
(a) als Wirkstoff ein natürliches Gemisch konjugierter equiner Oestrogene, das in Form eines pharmazeutisch präformulierten homogenen Trockenextraktes vorliegt, wobei der Trockenextrakt einen pro Menge Trägerstoff definierten, (bezogen auf die Haupthormon-Komponenten) standardisierten Wirkstoffgehalt des Gemisches natürlicher konjugierter equiner Oestrogene enthält, und der Wirkstoffgehalt durch Aufsprühen aus einer wäßrigen Lösung auf einen pulver- und/oder granulatförmigen pharmazeutischen Trägerstoff aus der Gruppe mikrokristalliner Cellulosen oder einem Gemisch von mikrokristalliner Cellulose mit Lactose und Trocknung aufgebracht wurde,
   und wenigstens einen gelbildenden pharmazeutischen polymeren Trägerstoff aus der Gruppe der Cellulose- und/oder Stärkederivate sowie gegebenenfalls einen oder mehrere weitere pharmazeutische Hilfsstoffe aus der Gruppe zusätzlicher Trägerstoffe in Form von mikrokristallinen Cellulosen, wasserlöslicher osmotischer Agenzien und gegebenenfalls Bindemittel und/oder Schmiermittel gleichzeitig oder in beliebiger Reihenfolge miteinander vermischt, gewünschtenfalls auch unter Zusatz einer Polymerlösung, vorzugsweise einer Hydroxypropylmethylcellulose-Lösung oder eines Hydroxypropylmethylcellulose Sols,
(b) die unter (a) erhaltene, gegebenenfalls bereits granulierte Mischung nachfolgend in einer Tablettiermaschine zu Matrixkernen verpreßt, und
(c) die unter (b) erhaltenen Matrixkerne mit einem Überzug aus einer Filmzusammensetzung beschichtet, die wenigstens einen hydrophoben pharmazeutischen Filmbildner und weiterhin gegebenenfalls einen pharmazeutischen Weichmacher und/oder Porenbildner umfaßt.

Die Herstellung der erfindungsgemäßen Matrixtabletten bzw. der Matrixtablettenkerne erfolgt, indem die Träger- und Hilfsstoffe zusammen mit dem hormonhaltigen Wirkstoff in einem geeigneten Mischer gemischt und ggf. in einem geeigneten Gerät, z.B. unter Zusatz von Polymer wie HPMC (als Lösung oder Sol, z.B. 1-5 Gew.-%ig) granuliert werden. Das Granulat wird mit der geeigneten Menge Schmiermittel in einem geeigneten Mischer gemischt und anschließend auf einer geeigneten Tablettiermaschine verpreßt.

Im Rahmen des erfindungsgemäßen Verfahrens zur Herstellung der erfindungsgemäßen Matrix-Filmtablette werden vorteilhaft Trockenextrakte als Präformulierungen eingesetzt, die das natürliche Gemisch konjugierter Oestrogene als homogen verteilte Wirkstoffkomponente auf festen Trägerstoffen enthalten, und die nachfolgend weiter beschrieben werden.

Die vorliegende Erfindung verwendet eine pharmazeutische Präformulierung in Form eines festen, freifließenden Trockenextraktes für die Tablettierung, die gekennzeichnet ist durch
(a) einen pro Menge Trägermaterial definierten, (bezogen auf die Haupthormon-Komponenten) standardisierten Wirkstoffgehalt eines Gemisches natürlicher konjugierter equiner Oestrogene, wobei
(b) der Wirkstoffgehalt durch Aufsprühen aus einer wäßrigen Lösung auf einen pulver- und/oder granulatförmigen pharmazeutischen Trägerstoff aus der Gruppe mikrokristalliner Cellulosen oder einem Gemisch von mikrokristalliner Cellulose mit Lactose und Trocknung aufgebracht ist.

Konjugierte equine Oestrogene sind ein Gemisch verschiedener konjugierter Formen von Oestrogenen, die aus dem Harn trächtiger Stuten erhalten werden. Die zwei prinzipiellen Hauptkomponenten sind Natriumestronsulfat und Natriumequilinsulfat. Eine dritte wesentliche Komponente ist das 17-α-Dihydroequilinsulfat. Daneben sind auch noch Natrium-17-α-Estradiolsulfat und Natrium-17-β-Dihydroequilinsulfat von Bedeutung. Konjugierte Oestrogene (CE) enthalten üblicherweise 52,5 bis 61,5 Gew.-% Natriumestronsulfat, 22,5 bis 30,5 Gew.-% Natriumequilinsulfat, 13,5 bis 19,5 Gew.-% Natrium-17-α-Dihydroequilinsulfat, 2,5 bis 9,5 Gew.-% Natrium-17-α-Estra-diolsulfat und 0,5 bis 4 Gew.-% Natrium-17-β-Dihydroequilinsulfat. Der Gesamtanteil an Natriumestronsulfat und Natriumequilinsulfat liegt üblicherweise im Bereich von 79,5 bis 88 Gew.-%. Der Gesamtgehalt an freien Oestrogenen wie Estron, Equilin und 17-α-Dihydroequilin beträgt üblicherweise nicht mehr als 1,3 Gew.-%. Die vorstehenden Prozent-Angaben beziehen sich auf den sogenannten "Labeled Content", wie dieser gemäß European Pharmacopoeia 2001 oder analog der USP (United States Pharmacopoeia) über gaschromatographische Profile, im Vergleich zu Referenzlösungen üblicherweise ermittelt und berechnet werden kann.

Der Wirkstoffgehalt der im Gemisch natürlicher konjugierter equiner Oestrogene enthaltenen Hormone wird üblicherweise auf die Haupthormon-Komponenten standardisiert, wobei in der Regel auf die Summe der drei Hauptkomponenten Estron, Equilin und 17-α-Dihydroequilin und gelegentlich aber auch auf die Summe aus diesen drei Hauptkomponenten und zusätzlich 17-α-Estradiol und 17-β-Dihydroequilin abgestellt wird (jeweils konjugierte und freie Hormone).

In zweckmäßigen Ausgestaltungen der vorliegenden Erfindung zeichnet sich die pharmazeutische Präformulierung dadurch aus, daß der Wirkstoffgehalt berechnet als Trockensubstanz (TS) eines das Gemisch natürlicher konjugierter equiner Oestrogene enthaltenden Extraktes aus dem Harn trächtiger Stuten (Gesamthormongehalt einschließlich der freien Oestrogene und sonstiger Feststoffe) bezogen auf die Menge des pharmazeutischen Trägerstoffes in der Präformulierung im Bereich von 0,25 bis 0,70 g TS/g Trägerstoff, vorzugsweise im Bereich von 0,28 bis 0,64 g TS/g Trägerstoff, liegt.

Wird der Wirkstoffgehalt (Gesamthormongehalt einschließlich der freien Oestrogene) der pharmazeutischen Präformulierung als Gemisch natürlicher equiner konjugierter Oestrogene (CE) bezogen auf die Menge des pharmazeutischen Trägerstoffes in der Präformulierung berechnet, so liegt der Wirkstoffgehalt im Bereich von 35 bis 100 mg CE/g Trägerstoff, vorzugsweise im Bereich von 43 bis 90 mg CE/g Trägerstoff.

Nach Trocknung der durch Aufsprühen des CE-Wirkstoffgehaltes aus einer wäßrigen Lösung auf den pulver- und/oder granulatförmigen pharmazeutischen Trägerstoff aus der Gruppe mikrokristalliner Cellulosen oder auf ein Gemisch von wenigstens einer dieser mikrokristallinen Cellulosen mit Lactose erhaltenen pharmazeutischen Präformulierung, kann diese herstellungsbedingt noch einen geringen Anteil an Restfeuchte aufweisen. Üblicherweise liegt der Restfeuchteanteil hierbei im Rahmen der bei den angewendeten Trocknungsverfahren üblichen Maximalwerte. So beträgt die Restfeuchte in der pharmazeutischen Präformulierung insbesondere maximal etwa 3,0 Gew.-%, vorzugsweise maximal etwa 1,0 Gew.-%, bezogen auf die gesamte Präformulierung als 100 Gew.-% (Summe aus dem als Trockensubstanz berechneten Wirkstoffgehalt, dem pharmazeutischen Trägerstoff und Berücksichtigung des Anteils an Restfeuchte).

Wird der Wirkstoffgehalt der erfindungsgemäß verwendeten pharmazeutischen Präformulierung als Gesamthormongehalt (Summe aller konjugierten und freien Hormone) berechnet, so liegt der Wirkstoffgehalt im Bereich von etwa 35 bis 100 mg pro 1 g des pharmazeutischen Trägerstoffes, vorzugsweise im Bereich von etwa 43 bis 90 mg pro 1 g des pharmazeutischen Trägerstoffes.

Zweckmäßige Ausführungsformen der erfindungsgemäß verwendeten pharmazeutischen Präformulierung zeichnen sich dadurch aus, daß im Wirkstoffgehalt die konjugierten Hormone (jeweils als Natriumsalz des Sulfatesters), insbesondere die konjugierten Haupthormone, in folgenden Anteilen enthalten sind: 52,5 bis 61,5 % Estron, 22,5 bis 30,5 % Equilin, 13,5 bis 19,5 % 17-α-Dihydroequilin, 2,5 bis 9,5 % Estradiol, 0,5 bis 4,0 % 17-β-Dihydroequilin.

Weiterhin liegt in vorteilhaften Varianten der erfindungsgemäß verwendeten pharmazeutischen Präformulierung der Summenanteil an freien Hormonen in der Präformulierung im Bereich von maximal etwa 2 bis 3 mg pro 1 g des pharmazeutischen Trägerstoffes. Vorzugsweise liegt der Anteil an freien Hormonen im Wirkstoffgehalt der Präformulierung bezogen auf den Gesamtgehalt an Hormonen (Summe aller konjugierten und freien Hormone) unter 5 Gew.-%. Je nach Aufarbeitung des zur Herstellung der erfindungsgemäß verwendeten pharmazeutischen Präformulierung eingesetzten hormonhaltigen wäßrigen Lösungsextraktes kann der auf den Gesamthormongehalt bezogene Anteil an freien Hormonen auch deutlich darunter liegen, z.B. unter 2 Gew.-%.

Es zeigte sich überraschenderweise, daß durch Aufsprühen eines aus PMU gewonnenen CE-Lösungsextraktes durch die Wirbelschichttechnik auf bestimmte pharmazeutische Trägerstoffe, wie mikrokristalline Cellulosen oder Gemische dieser mikrokristallinen Cellulosen mit Lactose, die konjugierten Hormone auf diese Trägerstoffe homogen aufgetragen werden können und daß sich das hierdurch erhaltene feste, freifließende Trockenextrakt vorteilhaft eignet, um feste galenische Formen, wie z.B. Tabletten, herzustellen. Insbesondere können die erfindungsgemäß verwendeten pharmazeutischen Präformulierungen in Form des Trockenextraktes homogen in eine Tablette, vorzugsweise in eine Matrix-Tablette, verteilt und verpresst werden, wobei erwünschte Freisetzungsprofile erzielt werden können. Überraschenderweise zeigte sich hierbei auch, daß durch die Wahl des pharmazeutischen Trägerstoffes in Abhängigkeit der Wasserlöslichkeit des Trägerstoffes oder Trägerstoff-Gemisches die Freisetzungsgeschwindigkeit von, in einer Matrix-Tablette verpresst vorliegenden konjugierten Hormonen vorteilhaft beeinflusst werden kann. Hierbei beeinflussen insbesondere die Art und Zusammensetzung des pharmazeutischen Trägerstoffes bzw. Trägerstoffgemisches, z.B. die Art und die Eigenschaften mikrokristalliner Zellulose und Lactose, die Partikelgrösse und die Porosität des Wirkstoffgranulates und die Partikelgrössenverteilung vorteilhaft die Qualität der Verpressbarkeit der erhaltenen pharmazeutischen Präformulierung und in der Folge das Freisetzungsprofil der konjugierten Hormone aus einer mittels dieser pharmazeutischen Präformulierung hergestellten Matrix-Tablette. Weiterhin können neben den vorstehend genannten ausgewählten pharmazeutischen Trägerstoffen oder Trägerstoff-Gemischen geringe Mengen weiterer üblicher Tablettier-Hilfstoffe bzw. Stabilisatoren in der erfindungsgemäß verwendeten pharmazeutischen Präformulierung in geringer Menge vorliegen, wodurch eine weitere Beeinflussung des Freisetzungsprofils der Hormone sowie deren Stabilität in der pharmazeutischen Präformulierung oder daraus hergestellten festen pharmazeutischen Zubereitungen wie Tabletten, insbesondere Matrix-Tabletten, ermöglicht wird. Solche Tabletttier-Hilfstoffe sind z.B. Füllstoffe, Sprengmittel, Zerfallsförderer- oder -beschleuniger, Trockenbindemittel, Trockenmittel oder Adsorptionsmittel, Gleitmittel (z.B. Fließregulierungs-, Schmier- oder Formtrennmittel).
Diese beispielhaft genannten Tablettier-Hilfsstoffe, oder auch weitere dem Fachmann geläufige und üblicherweise in der Tablettenherstellung verwendete Hilfsstoffe, können zu den erfindungsgemäß verwendeten Präformulierungen maximal in solchen Mengen zugemischt werden, in denen sie auch in der fertigen Matrixtablette vorliegen sollen.

Die erfolgreiche Verwendbarkeit der erfindungsgemäß eigesetzten Präformulierung zur Herstellung von festen galenischen Formen natürlicher Gemische konjugierter equiner Oestrogen, insbesondere z.B. von Tabletten oder vorzugsweise Matrix-Tabletten, stellt einen wesentlichen Teilschritt bei der Herstellung der eigentlichen festen galenischen Form für die therapeutische bzw. prophylaktische Verabreichung an Patienten dar und beruht neben anderen Faktoren auch auf der Art der ausgewählten pulver- und/oder granulatförmigen pharmazeutischen Trägerstoffe, nämlich insbesondere pharmazeutische Trägerstoffe aus der Gruppe mikrokristalliner Cellulosen und die fakultativ im Gemisch mit mikrokristalliner Cellulose eingesetzte Lactose. Ist der pharmazeutische Trägerstoff in der erfindungsgemäß verwendeten pharmazeutischen Präformulierung eine mikrokristalline Cellulose, so kann diese eine einzelne Type mikrokristalliner Cellulose sein oder auch ein Gemisch verschiedener Typen mikrokristalliner Cellulosen. Eine andere Variante der Erfindung enthält Gemische von mikrokristalliner Cellulose mit Lactose, die jeweils in pulver- und/oder granulatförmiger Form vorliegen. In der Variante der erfindungsgemäß verwendeten Präformulierungen, in der Gemische aus einer mikrokristallinen Cellulose mit Lactose als Trägerstoff vorliegen, kann deren Mischungsverhältnis in weiten Bereichen variiert werden, wobei jedoch zweckmäßigerweise darauf zu achten ist, daß die Menge der mikrokristalliner Cellulose nicht unter 60 Gew.-%, vorzugsweise nicht unter 80 Gew.-%, und die Menge der Lactose nicht über 40 Gew.-%, vorzugsweise nicht über 20 Gew.-%, liegen sollte. Zweckmäßige Mischungsverhältnisse von mikrokristalliner.Cellulose zu Lactose sind gegeben, sofern das Gewichtsverhältnis von mikrokristalliner Cellulose zu Lactose im Bereich von 8:2 bis 6:4 liegt, vorzugsweise im Bereich von 7,5:2,5 bis 6,5:3,5. In einer beispielhaften Ausgestaltung der erfindungsgemäß verwendeten Präformulierung beträgt das Mischungsverhältnis von mikrokristalliner Cellulose zu Lactose etwa 7:3 als Gewichtsverhältnis.

Mikrokristalline Cellulosen sind als pharmazeutischer Grundstoff in verschiedenen Ausführungen im Handel erhältlich, z.B. als Avicel^{®} (z.B. der Firma Lehmann & Voss & Co., Hamburg, Deutschland), insbesondere als Avicel^{®}-Typen PH 101, PH 102, PH 102 SCG oder PH 103. Die als Avicel^{®} im Handel erhältlichen mikrokristallinen Cellulosen für pharmazeutische Zwecke weisen üblicherweise z.B. folgende allgemeine Spezifikation auf: Wassergehalt unter 5 Gew.-% (Type PH 103: unter 3 Gew.-%); Asche unter 10; Brechungsindex 1,55; pH (Dispersion) 5,5 bis 7,0; mittlere Korngrößen für

| Type | PH 101 | PH 102 | PH 102 SCG | PH 103 |
|---|---|---|---|---|
| | 50 µm | 100 µm | 130 µm | 50 µm; |

und ein Teilchengrößenverteilung von:

| Type | PH 101 | PH 102 | PH 102 SCG | PH 103 |
|---|---|---|---|---|
| 250 µm | < 1 % | < 8 % | < 8 % | < 1 % |
| 150 µm | | | > 23 % | |
| 75 µm | < 30 % | > 45 % | > 63 % | < 30 % |

Eine weitere, erfindungsgemäß verwendbare, im Handel erhältliche mikrokristalline Cellulose für pharmazeutische Zwecke wird unter dem Handelsnamen Vivapur^{®}, z.B als Type Vivapur^{®} 101 oder Vivapur^{®} 12, vertrieben (z.B. von der Firma J. Rettenmaier & Söhne GmbH + Co, Rosenberg, Deutschland). Vivapur^{®} 101 weist üblicherweise z.B. folgende allgemeine Spezifikation auf: Trocknungsverlust maximal 6 Gew.-%; Polymerisationsgrad (Identität) < 350; Schüttdichte 0,26 bis 0,32 g/ml; Korngrößenverteilung: d₁₀: < 30 µm, d₅₀: 40 bis 70 µm, d₉₀: > 80 µm; Siebanalyse (Rückstand auf dem Luftstrahlsieb): > 250 µm maximal 1 Gew.-%, > 75 µm maximal 30 Gew.-%, > 32 µm mindestens 50 Gew.-%. pH 5,0 bis 7,0; Sulfatasche maximal 0,05 Gew.%. Vivapur^{®} 12 weist üblicherweise z.B. folgende allgemeine Spezifikation auf: Trocknungsverlust maximal 6 Gew.-%; Schüttdichte etwa 0,35 g/ml; Stampfvolumen etwa 1,9 ml/g; mittlere Korngröße 160 µm; Korngrößenverteilung: d₁₀: < 30 µm, d₅₀: 40 bis 70 µm, d₉₀: > 80 µm; Siebanalyse (Rückstand auf dem Luftstrahlsieb): 400 µm maximal 1 Gew.-%, 160 µm maximal 50 Gew.-%, 50 µm mindestens 70 Gew.-%.

Lactose ist ebenfalls als pharmazeutischer Grundstoff im Handel als ein weißes, gesiebtes, kristallines, geruchloses, in Wasser leicht und in Ethanol praktisch unlösliches Pulver erhältlich, z.B. als Capsulac^{®} (der Firma Meggle), insbesondere als Capsulac^{®} 60 oder Capsulac^{®} 200. Die im Handel als Capsulac^{®} 60 erhältliche Lactose für pharmazeutische Zwecke weist üblicherweise folgende Spezifikation auf: sauer oder alkalisch reagierende Substanzen maximal 0,4 ml 0,1 n Natronlauge; spezifische Drehung 54,4° bis 55,9°; Wasser (DAB) 4,5 bis 5,5 Gew.-%; Trocknungsverlust maximal 0,5 Gew.-%; Sulfatasche maximal 0,1 Gew.-%; Glührückstand maximal 0,1 Gew.-%; Korngrößenverteilung (Rüttelsiebung, 25 g, 10 Minuten): < 100 µm maximal 10 Gew.-%, < 630 µm mindestens 97 Gew.-%. Die im Handel als Capsulac^{®} 200 (Type EP D 80) erhältliche Lactose für pharmazeutische Zwecke weist üblicherweise folgende Spezifikation auf: sauer oder alkalisch reagierende Substanzen maximal 0,19 ml 0,1 n Natronlauge; spezifische Drehung 55,4°; Gesamtwasser 5,39 Gew.-%; Trocknungsverlust 0,17 Gew.-%; Sulfatasche 0,04 Gew.-%; Glührückstand 0,04 Gew.-%; Korngrößenverteilung (Luftstrahlsiebung, 10 g, 2 Minuten): < 32 µm 45 bis 75 Gew.-%, < 100 µm mindestens 90 Gew.-%.

In zweckmäßigen Ausgestaltungen können die erfindungsgemäß verwendeten Präformulierungen durch weitere Parameter charakterisiert werden, wie z.B. die Partikelgrößenverteilung, die mittlere bzw. durchschnittliche Partikelgröße, die Porosität der Partikel, das mittlere Schüttgewicht (Schüttdichte) und/oder mittlere Schüttvolumen.

Zweckmäßige erfindungsgemäß verwendbare pharmazeutische Präformulierungen weisen z.B. ein mittleres Schüttvolumen im Bereich von 1,8 bis 3,0 ml/g auf. Das mittlere Schüttgewicht (Schüttdichte) der erfindungsgemäß verwendeten pharmazeutischen Präformulierung liegt z.B. im Bereich von 0,3 bis 0,6 g/ml. In einer Alternative zeichnet sich die erfindungsgemäß verwendete pharmazeutische Präformulierung dadurch aus, daß die Präformulierung eine durch Siebanalyse als prozentuale Durchgangssumme in Abhängigkeit von der Siebmaschenweite charakterisierte Partikelgrößenverteilung von 100 Gew.-% der Partikel bei einer Maschenweite von 500 µm, von wenigstens 98 Gew.-% der Partikel bei einer Maschenweite von 250 µm, von etwa 65 bis 99,5 Gew.-% der Partikel bei einer Maschenweite von 160 µm, von etwa 35 bis 87 Gew.-% der Partikel bei einer Maschenweite von 125 µm, und einem Feinanteil von unter 23 Gew.-% bei einer Maschenweite von 63 µm, jeweils bezogen auf die Gesamtsumme der Siebfraktionen als 100 Gew.-%, aufweist. Alternativ zeichnet sich die erfindungsgemäß verwendete pharmazeutische Präformulierung dadurch aus, daß die Präformulierung eine durch Siebanalyse in Abhängigkeit von der Siebmaschenweite charakterisierte Partikelgrößenverteilung von etwa 0,15 bis maximal 2 Gew.-% der Partikel größer als eine Maschenweite von 250 µm, von etwa 3 bis 31 Gew.-% der Partikel größer als eine Maschenweite von 160 µm, von etwa 8 bis 36 Gew.-% der Partikel größer als eine Maschenweite von 125 µm, und einem Feinanteil der Partikel von etwa 3 bis maximal 23 Gew.-% bei einer Maschenweite von 63 µm, jeweils bezogen auf die Gesamtsumme der Siebfraktionen als 100 Gew.-%, aufweist. Die mittlere (durchschnittliche) Partikelgröße der erfindungsgemäß verwendeten pharmazeutischen Präformulierung liegt zweckmäßigerweise im Bereich von 50 bis 250 µm, vorzugsweise im Bereich von 75 bis 150 µm.

Die vorliegende Erfindung beschreibt weiterhin auch ein Verfahren zur Herstellung der vorstehend beschriebenen erfindungsgemäß verwendeten Trockenextrakte von natürlichen Gemischen konjugierter equiner Oestrogene, insbesondere von Gemischen aus dem Harn trächtiger Stuten gewonnener, konjugierter Oestrogene, wobei durch diese Trockenextrakte pharmazeutische Präformulierungen von natürlicher Gemischen konjugierter Oestrogene bereitgestellt werden, die sich für die Herstellung von festen galenischen Formen, z.B. zur Herstellung von Tabletten und insbesondere gewünschtenfalls auch für Direkttablettierung, eignen. Das Verfahren zur Herstellung der erfindungsgemäß verwendeten pharmazeutischen Präformulierung in Form eines festen, freifließenden Trockenextraktes der oben definierten Art für die Tablettierung zeichnet sich dadurch aus, daß man auf einen, in einem Wirbelschichtgerät fluidisierten pulver- und/oder granulatförmigen pharmazeutischen Trägerstoff, der aus der Gruppe mikrokristalliner Cellulosen oder einem Gemisch von mikrokristalliner Cellulose mit Lactose ausgewählt ist, eine wäßrige Lösung, die ein Gemisch natürlicher konjugierter equiner Oestrogene als Wirkstoff enthält, in einer Menge aufsprüht, die dem in der pharmazeutischen Präformulierung erwünschten definierten, (bezogen auf die Haupthormon-Komponenten) standardisierten Wirkstoffgehalt entspricht, und man die erhaltenen wirkstoffhaltigen Partikel trocknet.

Die im Verfahren einsetzbaren mikrokristallinen Cellulose-Typen und Lactose-Typen sind weiter oben bereits im Zusammenhang mit den erfindungsgemäß verwendeten pharmazeutischen Präformulierungen beschrieben.

Für das Verfahren zur Gewinnung der Trockenextrakte können CE-enthaltende aus PMU beliebiger Herkunft gewonnene wäßrige Lösungsextrakte in einem weiten Bereich varierender CE-Konzentration eingesetzt werden, die durch die weiter oben im Stand der Technik beschriebenen Aufarbeitungsverfahren für PMU erhalten werden können, insbesondere durch das in der WO 98/08526 beschriebene oder ähnlicher Verfahren unter Verwendung semipolarer, vorzugsweise nicht-ionischer semipolarer Adsorberharze. Je nach Konzentration der CE und der ggf. in diesen Extrakten verbliebenen Begleitstoffe können diese wäßrigen Extrakte durch weiteres Entfernen von Lösungsmittel aufkonzentriert oder durch Zugabe von weiterem Wasser bzw. von mit Wasser mischbaren organischen Lösungsmitteln wie z.B. niederen aliphatischen Alkoholen auf gewünschte Wirkstoffgehalte für den Einsatz im vorliegenden Verfahren zur Herstellung der verwendeten Trockenextrakte eingestellt werden.

In einer Variante des Verfahrens zur gewinnung der Trokkenextrakte kann die eingesetzte Wirkstoff enthaltende wäßrige Lösung somit neben dem Wasser noch andere, mit Wasser mischbare organische Lösungsmittel, insbesondere noch einen oder mehrere niedere aliphatische Alkohole, als zusätzliches Lösungsmittel enthalten. Geeignete niedere aliphatische Alkohole sind insbesondere solche mit ein bis vier Kohlenstoffatomen, beispielsweise Methanol, Ethanol, Isopropanol oder n-Butanol. Bevorzugt sind Methanol, Ethanol oder Isopropanol. Die organischen Lösungsmittel, insbesondere die Alkohole, können auch im Gemisch miteinander als zusätzliches Lösungsmittel der wäßrigen Lösung zugesetzt sein. Die Menge des mit Wasser mischbaren organischen Lösungsmittelanteils, insbesondere des Alkoholanteils, kann in der wäßrigen Lösung in den Bereichen liegen, wie diese in der WO 98/08526 als geeignet beschrieben sind. Andere ggf. geeignete mit Wasser mischbare Lösungsmittel wie Ketone oder wasserlösliche Ether sind ebenfalls in der WO 98/08526 beschrieben.

Bevorzugt werden im Verfahren zur Herstellung der verwendeten Trockenextrakte allerdings Wirkstoff enthaltende wäßrige Lösungen, d.h. CE-Extraktlösungen oder -konzentrate, eingesetzt, die eine weitgehend von organischem Lösemittel befreite, zur galenischen Weiterverarbeitung geeignete wäßrige Lösung, d.h. eine weitgehend rein wäßrige Lösung, der CE oder ein weitgehend von organischem Lösemittel befreites Konzentrat der CE sind. Sehr bevorzugt sind hierbei rein wäßrige Lösungen oder Konzentrate des natürlichen Gemisches konjugierter Oestrogene.

Zweckmäßige Ausführungsvarianten des vorliegenden Verfahren zur Herstellung der verwendeten Trockenextrakte zeichnen sich dadurch aus, daß die eingesetzte wäßrige Lösung einen Wirkstoffgehalt berechnet als Trockensubstanz des Gemisches natürlicher equiner konjugierter Oestrogene (Gesamthormongehalt einschließlich der freien Oestrogene und sonstiger Feststoffe) im Bereich von etwa 3,5 bis 20 Gew.-% bezogen auf die wäßrige Lösung als 100 Gew.-% aufweist. Bevorzugt liegt der als Trockensubstanz des natürlichen Gemisches konjugierter equiner Oestrogene berechnete Wirkstoffgehalt in der wäßrigen Lösung im Bereich von etwa 3,5 bis 14,5 Gew.-% bezogen auf die wäßrige Lösung als 100 Gew.-%. Wird der Wirkstoffgehalt der im Verfahren zur Herstellung der Trockenextrakte eingesetzten wäßrigen Lösung als Gesamthormongehalt (einschließlich der freien Oestrogene) berechnet, so weist die eingesetzte wäßrige Lösung einen Wirkstoffgehalt im Bereich von 10 bis 100 mg pro 1 g der wäßrigen Lösung, vorzugsweise im Bereich von 10 bis 40 mg pro 1 g der wäßrigen Lösung, auf.

Wird im Verfahren zur Herstellung der Trockenextrakte als wäßrige Lösung ein Konzentrat eingesetzt, so weist dieses zweckmäßigerweise einen Wirkstoffgehalt berechnet als Trockensubstanz des Gemisches natürlicher konjugierter equiner Oestrogene (Gesamthormongehalt einschließlich der freien Oestrogene und sonstiger Feststoffe) im Bereich von größer 20 Gew.-% bezogen auf das Konzentrat als 100 Gew.-% auf. Wird der Wirkstoffgehalt des im Verfahren zur Herstellung der Trockenextrakte eingesetzten wäßrigen Konzentrates als Gesamthormongehalt (einschließlich der freien Oestrogene) des Gemisches natürlicher equiner konjugierter Oestrogene (CE) berechnet, so weist das eingesetzte Konzentrat zweckmäßigerweise einen Wirkstoffgehalt von größer 40 mg pro 1 g des Konzentrates (100 Gew.-%) auf.

Zweckmäßigerweise werden im Verfahren zur Herstellung der Trockenextrakte wäßrige Lösungen eingesetzt, in denen der Gesamthormongehalt (einschließlich der freien Oestrogene) bezogen auf die in der wäßrigen Lösung enthaltene Trockensubstanz als 100 Gew.-% im Bereich von 18 bis 31 Gew.-% liegt.

Das Verfahren zur Herstellung der erfindungsgemäß verwendeten Trockenextrakte bzw. Präformulierungen von natürlichen Gemischen konjugierter Oestrogene, insbesondere von Gemischen aus dem Harn trächtiger Stuten gewonnener, konjugierter Oestrogene kann in an sich beliebigen, üblichen Wirbelschichttrocknungsgeräten, insbesondere solchen für den Einsatz im pharmazeutischen Betrieb, durchgeführt werden. Geeignete Wirbelschichtgeräte sind z.B. das Wirbelschichtgerät "Strea I". Im Verfahren zur Herstellung der Trockenextrakte wird der pulver- oder granulatförmige pharmazeutische Trägerstoff, z.B. die mikrokristalline Cellulose oder ein Gemisch von mikrokristalliner Cellulose mit Lactose, im Wirbelschichtgerät in einer vorberechneten Produktionsmenge vorgelegt und mittels eines Luftstromes fluidisiert. Anschließend wird eine ein natürliches Gemisch konjugierter Oestrogene als Wirkstoff enthaltende wäßrige Lösung in einer Menge, die dem in der Präformulierung erwünschten Wirkstoffgehalt entspricht, auf den Trägerstoff aufsprüht und die erhaltenen wirkstoffhaltigen Partikel trocknet.

Das Verfahren kann hierbei sowohl kontinuierlich als auch diskontinuierlich im Chargenbetrieb durchgeführt sowie neben der Art und Menge des eingesetzten Trägerstoffes bzw. neben der Art, der Menge und dem wirkstoffgehalt der eingesetzten wäßrigen Lösung, weiterhin über dem Fachmann in der Wirbelschichttechnik geläufige Verfahrensparameter wie z.B. Zu- und Ablufttemperatur, Menge des zu- und abgeführten Luftstromes, die Aufsprühgeschwindigkeit der wäßrigen Lösung sowie bei kontinuierlicher Verfahrensweise auch durch die Geschwindigkeit des Feststoffeintrages und Produktaustrages bzw. der Verweildauer des Produktes im Wirbelschichtgerät gesteuert werden.

In einer zweckmäßigen Variante des Verfahrens zur Herstellung der Trockenextrakte liegt z.B. die anhand der Ablufttemperatur regulierte Temperatur des im Wirbelschichtgerät fluidisierten Präformulierungs-Produktes im Bereich von 25 bis 60 °C, vorzugsweise im Bereich von 45 bis 55 °C. In einem Ausführungsbeispiel des Verfahrens zur Herstellung der Trockenextrakte liegt die anhand der Ablufttemperatur regugulierte Temperatur des im Wirbelschichtgerät fluidisierten Präformulierungs-Produktes bei etwa 45 bis 55 °C.

In einer zweckmäßigen Variante des Verfahrens zur Herstellung der Trockenextrakte liegt z.B. die über die relative Abluftfeuchte regulierte Prozeßfeuchtigkeit im Wirbelschichtgerät im Bereich von 50 bis 80 % r.h. (r.h. = relative humidity bzw. relative Feuchte).

In einer zweckmäßigen Variante des Verfahrens zur Herstellung der Trockenextrakte wird z.B. die eingesetzte Wirkstoff enthaltende wäßrige Lösung mit einer Sprühgeschwindigkeit von 20 bis 50 g/min, auf den im Wirbelschichtgerät fluidisierten pulver- und/oder granulatförmigen pharmazeutischen Trägerstoff aufgesprüht.

Im Verfahren zur Herstellung der erfindungsgemäß verwendeten Trockenextrakte bzw. Präformulierungen von natürlichen Gemischen konjugierter Oestrogene, insbesondere von Gemischen aus dem Harn trächtiger Stuten gewonnener, konjugierter Oe strogene werden in zweckmäßigen Ausführungsvarianten pulver- und/oder granulatförmige Trägerstoffe eingesetzt, die durch bestimmte Partikeleigenschaften gekennzeichnet sind und somit zur gezielten Steuerung der Partikeleigenschaften des Trockenextrakt- bzw. Präformulierungsproduktes verwendet werden können. Als geeignete Parameter für die Partikeleigenschaften der eingesetzten pulver- bzw. granulatförmigen Trägerstoffe können ebenso wie für die Charakterisierung der auf dieser Basis hergestellten Trockenextrakt- bzw. Präformulierungsprodukte z.B. die Partikelgrößenverteilung, die mittlere bzw. durchschnittliche Partikelgröße, die Porosität der Partikel oder das mittlere Schüttgewicht sowie vom Fachmann im konkreten Fall als zweckmäßig erachtete weitere Parameter herangezogen werden. Nachfolgend werden zur Orientierung einige zweckmäßige Bereiche dieser Partikelparameter angegeben.

In einer vorteilhaften Variante des Verfahrens zur Herstellung der Trockenextrakte wird ein pulver- und/oder granulatförmiger pharmazeutischer Trägerstoff, insbesondere eine mikrokristalline Cellulose, eingesetzt, der eine durch Siebanalyse als prozentuale Durchgangssumme in Abhängigkeit von der Siebmaschenweite charakterisierte Partikelgrößenverteilung von 100 Gew.-% der Partikel bei einer Maschenweite von 500 µm, von wenigstens 99 Gew.-% der Partikel bei einer Maschenweite von 250 µm, von etwa 85 bis 95 Gew.-% der Partikel bei einer Maschenweite von 160 µm, von etwa 70 bis 80 Gew.-% der Partikel bei einer Maschenweite von 125 µm, und einem Feinanteil von bis zu etwa 50 Gew.-% bei einer Maschenweite von 63 µm, jeweils bezogen auf die Gesamtsumme der Siebfraktionen als 100 Gew.-%, aufweist. Besonders zweckmäßige im Verfahren zur Herstellung der Trockenextrakte eingesetzte pulver- und/oder granulatförmige pharmazeutische Trägerstoffe, insbesondere die mikrokristalline Cellulose, weisen hierbei eine mittlere (durchschnittliche) Partikelgröße im Bereich von 50 bis 130 µm auf. Der im Verfahren eingesetzte pulver- und/oder granulatförmige pharmazeutische Trägerstoff, insbesondere die mikrokristalline Cellulose, weist z.B. ein Schüttgewicht (Schüttdichte) im Bereich von etwa 25 bis 35 g/ml auf. Weiterhin sind die im Verfahren eingesetzten pulver- und/oder granulatförmigen pharmazeutischen Trägerstoffe, insbesondere die mikrokristalline Cellulose, **dadurch gekennzeichnet, daß** der Wassergehalt (Trocknungsverlust) maximal etwa 6 Gew.-% beträgt.

Nach dem Verfahren zur Herstellung der Trockenextrakte wird in vorteilhafter Weise ein für die Herstellung von Pharmazeutika, die das natürliche Gemisch konjugierter Oestrogene aus dem PMU als Wirkkomponente enthalten, dienendes Ausgangsmaterial bereitgestellt, das sich als Trockenextrakt bzw. Präformulierung von hervorragender Qualität vorteilhaft für die Weiterverarbeitung durch Direkttablettierung eignet.

Das Verfahren zur Herstellung der Trockenextrakte und die erfindungsgemäß verwendete Präformulierung weisen eine Reihe von Vorteilen insbesondere auch gegenüber anderen Verfahrensweisen auf. Es können CE-enthaltende wäßrige Extrakte mit geringer Hormonkonzentration verarbeitet werden. Im Gegensatz zu Beobachtungen bei konventioneller Sprühtrockung solcher CE-enthaltender Extrakte werden beim Verfahren zur Herstellung der Trockenextrakte in der Wirbelschicht unerwünschte Anhaftungen z.B. an den Düsen nicht beobachtet. Die thermische Belastung der wertvollen Hormonbestandteile der eingesetzten wäßrigen Extrakte ist beim Verfahren zur Herstellung der Trockenextrakte in der Wirbelschicht sehr gering. Klebrige Eigenschaften, z.B. Verklumpung, des CE-enthaltenden wäßrigen Extraktes kommen weniger zum Tragen als bei anderen Trocknungsverfahren wie z.B. bei Single-Pot Technologie. Gegenüber Arbeitsweisen in Vakuumtrocknern etc. ist das Verfahren zur Herstellung der Trockenextrakte ein kontinuierlich durchführbares Verfahren, daß zudem - sowohl bei kontinuierlicher als auch diskontinuierlicher Verfahrensweise - den Auftrag großer Flüssigkeitsmengen, auch ohne Überfeuchtung, erlaubt. Im Verfahren zur Herstellung der Trockenextrakte kann ein weites Spektrum an Extrakten sowohl hinsichtlich der Hormonkonzentration als auch der.Konzentration an Begleitstoffen verarbeitet werden. Dadurch kann das Verfahren sehr gut die aufgrund der natürlichen Schwankungen des PMU-Ausgangsmaterials in der großtechnischen Praxis zu bewältigenden Probleme lösen. Es zeigte sich, daß durch Aufsprühen eines Hormonkonzentrates mit Hilfe der Wirbelschichttechnik auf erfindungsgemäß verwendete Trägerstoffe, wie mikrokristalline Cellulose oder ggf. Gemische von mikrokristalliner Cellulose mit Lactose, die konjugierten Hormone homogen auf die Trägerstoffe aufgetragen werden können. Die nach dem Verfahren hergestellten Präformulierungen in Form fester, freifließender Trockenextrakte sind sehr stabile pulver- bzw. partikelförmige hormonhaltige Produkte, die überraschend gut in Matrixtabletten homogen verteilt und verpreßt werden können. Somit sich lassen sich aus den erfindungsgemäß verwende ten pharmazeutischen Präformulierungen in einfacher Weise Matrixtabletten mit einem gewünschten Freisetzungsprofil fertigen.

Der erfolgreiche erfindungsgemäße Einsatz der Präformulierungen in der Tablettierung, beispielsweise Direkttablettierung, ggf. auch unter vorheriger Granulierung (z.B. mit Hydroxypropylmethylcellulose-Lösung oder -Sol), stellt einen wesentlichen Beitrag zur Herstellung einer geeigneten festen galenischen Form für die therapeutische bzw. prophylaktische Verabreichung an Patienten dar. Hierbei ist auch die Art der für die Herstellung der Präformulierung gewählten pulver- oder granulatförmigen pharmazeutischen Trägerstoffe für die konjugierten Oestrogene, nämlich insbesondere pharmazeutische Trägerstoffe aus der Gruppe mikrokristalliner Cellulosen und fakultativ im Gemisch mit mikrokristalliner Cellulose eingesetzte Lactose, für die Qualität der erfindungsgemäßen Matrix-Filmtablette von Bedeutung. Bevorzugt sind als Trägerstoffe für die konjugierten Oestrogene in der Präformulierung insbesondere Gemische von mikrokristalliner Cellulose mit Lactose, die jeweils in pulver- oder granulatförmiger Form vorliegen. Bei bevorzugten Gemischen aus einer mikrokristallinen Cellulose mit Lactose als Trägerstoff kann deren Mischungsverhältnis in weiten Bereichen variiert werden. Zweckmäßige Mischungsverhältnisse von mikrokristalliner Cellulose zu Lactose sind oben angegeben. In einer beispielhaften Ausgestaltung der erfindungsgemäß zur Herstellung von Matrix-Filmtabletten eingesetzten Präformulierung beträgt das Mischungsverhältnis von mikrokristalliner Cellulose zu Lactose etwa 7:3 als Gewichtsverhältnis.

Mikrokristalline Cellulosen sind als pharmazeutischer Grundstoff in verschiedenen Ausführungen im Handel erhältlich und oben beschrieben, z.B. als Avicel^{®} oder Avicel^{®} PH 102. Lactose ist ebenfalls als pharmazeutischer Grundstoff im Handel erhältlich, z.B. als Capsulac^{®}, insbesondere als Capsulac^{®} 60, und wurde ebenfalls bereits oben beschrieben.

Die erfindungsgemäßen Matrix-Filmtabletten können unter Berücksichtigung der vorstehenden Angaben unter Anwendung an sich üblicher galenischer Verfahrensweisen zur Herstellung von Matrix-Tablettenkernen, z.B. durch Tablettierung, beispielsweise Direkttablettierung, zur nachfolgenden Beschichtung der Matrix-Tablettenkerne mit einem Filmüberzug und schließlich gegebenenfalls zum Aufbringen einer Zuckerdragierung gefertigt werden. Die Matrix-Tablettekerne könne mit dem Filmüberzug versehen werden, indem in einem geeigneten Gerät eine Suspension aus z.B. Polymethacrylat und/oder Polymethacrylat-Derivaten (z.B. Eudrojet, RL 30D), z.B. PEG 6000, z.B. Triethylcitrat, Talkum und ggf. Hydroxypropylmethylcellulose auf den Matrixkern aufgetragen wird. Abschließend kann auf die mit dem Filmüberzug versehenen Matrix-Filmtabletten noch zusätzlich eine Zuckerdragierung in üblicher galenischer Verfahrensweise aufgebracht werden.

Die nachfolgenden Beispiele sollen die Erfindung näher erläutern, ohne jedoch ihren Umfang zu beschränken.

### Beispiele

### Beispiel 1:

### Trocknung bzw. Herstellung einer Präformulierung mit hormonhaltigem Wirkstoff in einem Wirbelschichtgerät, und Hormonbilanz

Es wurden eine Reihe von Versuchen mit dem Ziel der Entwicklung eines hormonhaltigen Wirkstoffs durch Trocknung der Hormone aus Urinkonzentrat von trächtigen Stuten durchgeführt. Die konjugierten Hormone mußten hierbei in eine Form gebracht werden, welche die chemische Stabilität der Hormone gewährleistet und die Verarbeitung der Hormone in einer Tablette ermöglicht. Es wurde ein Urinkonzentrat (konzentrierte wäßrige Lösung von Urin trächtiger Stuten = PMU) aus einer Sammelkampagne in Asien eingesetzt, das durch seine Menge an Trockensubstanz und Hormonkonzentration charakterisiert war. Das Urinkonzentrat wurde vor der Verwendung gemäß dem Verfahren der WO 98/08526 aufgearbeitet, um unerwünschte Begleitstoffe wie Harnstoff, HPMF und Kresole abzutrennen.

In den Versuchen zeigte sich, daß durch Aufsprühen des Hormonkonzentrates durch die Wirbelschichttechnik auf Trägerstoffe wie mikrokristalline Cellulose oder Gemische aus mikrokristalliner Cellulose mit Lactose die konjugierten Hormone auf die Hilfsstoffe homogen aufgetragen werden konnten.

Ein aus einer Sammelkampagne bereitgestelltes Urinkonzentrat wurde auf mikrokristalline Cellulose oder auf ein Gemisch von mikrokristalliner Cellulose und Lactose aufgesprüht und dadurch die Hormone auf den Träger oder das Trägerstoffgemisch aufgetragen. Dieser Prozeß wurde in einem Wirbelschichtgranulator durchgeführt. Die Partikelgröße und Porosität des Wirkstoffgranulats wurde durch die Zuluft- und Ablufttemperatur und die Sprühgeschwindigkeit reguliert. Als Parameter für den Prozeß dienten die Produkttemperatur (reguliert an Hand der Ablufttemperatur), die im Bereich von 25 bis 55 °C eingestellt war, sowie die Prozeßfeuchtigkeit (reguliert über relative Abluftfeuchte), die im Bereich von 50 bis 80 % relative Luftfeuchte eingestellt war. Die Sprühgeschwindigkeit wurde entsprechend gewählt, um die vorgenannten Bereiche einzuhalten.

Zur Herstellung von Trockenextrakten von natürlichen Gemischen konjugierter Oestrogene wurde in diesen Versuchen ein Wirbelschichtgerät (Strea 1) eingesetzt, mit dem ca. 1 kg Trockenextrakt pro Ansatz hergestellt werden kann. Der wäßrige, ein natürliches Gemisch konjugierter Oestrogene enthaltender Lösungsextrakt wurde nach dem Top-Spray-Verfahren in das Wirbelschichtgerät eingebracht. Die weitere technische Ausrüstung umfaßte:
- Sartorius Waage / 6,2 kg / Typ LC6200S-OD2,
- Schlauchpumpe Masterflex 07523-27 mit Pumpenkopf 7518-10,
- Feuchtemeßgerät vom Typ HR 73 von Mettler Toledo.

Die Versuche im Wirbelschichtgerät wurden mit wäßrigen, ein natürliches Gemisch konjugierter Oestrogene enthaltenden Lösungsextrakten durchgeführt, die aus einer gemäß dem in der WO 98/08526 beschriebenen Verfahren aufgearbeiteten Sammelkampagne in Asien entstammten, wobei die hormonhaltigen wäßrigen Extrakte die folgenden Hormongehalte aufwiesen:
Versuch 1: TS = 9,2 Gew.-%
Versuch 2: TS = 15,9 Gew.-%
Versuch 3: TS = 19,3 Gew.-%
Versuch 4: TS = 9,2 Gew.-%

In weiteren Versuchen wurden CE-enthaltende wäßrige Lösungsextrakte mit TS = 11,8 Gew.-% (Versuch 5) bzw. TS = 9,9 Gew.-% (Versuch 6) eingesetzt. Die wäßrigen Lösungsextrakte wiesen alle einen kristallinen oder öligen Niederschlag auf, der eine homogene Verarbeitung aber nicht wesentlich beeinträchtigte. Die wäßrigen Lösungsextrakte besaßen nur einen relativ niedrigen Hormongehalt, weshalb die Trockenextrakte auf einen theoretischen Soll-Gehalt von 45 mg konjugierte Oestrogene pro g Trockenextrakt eingestellt wurden.

Als Trägerstoffe für das natürliche Gemisch konjugierter Oestrogene wurden eingesetzt:
- Avicel PH 102,
- Capsulac 60.

### VERSUCHSDURCHFÜHRUNG

Herstellung eines Trockenextraktes mit einem Gehalt von 45 mg konjugierter Oestrogene pro g Trockenextrakt bei Vorlagen von 570 bis 680 g des Trägerstoffes.

### Versuch 1:

| | |
|---|---|
| eingesetzter Extrakt: | 4023,1 g; TS = 9,2 Gew.-%; |
| | Dichte: 1,0365 g/l; CE = 12,14 g/l |
| Vorlage: | 677,0 g Avicel PH 102 |
| Sprührate: | 40-50 g/min (ungefährer Mittelwert) |
| relative Abluftfeuchte: | 70-80 % |
| Ablufttemperatur: | 32-34 °C |

### Versuch 2:

| | |
|---|---|
| eingesetzter Extrakt: | 2400,0 g; TS = 15,9 Gew.-%; |
| | Dichte: 1,0662 g/l; CE = 20,86 g/l |
| Vorlage: | 661,9 g Avicel PH 102 |
| Sprührate: | 40-50 g/min (ungefährer Mittelwert) |
| relative Abluftfeuchte: | 70-80 % |
| Ablufttemperatur: | 32-34 °C |

### Versuch 3:

| | |
|---|---|
| eingesetzter Extrakt: | 1904,6 g; TS = 19,3 Gew.-%; |
| | Dichte: 1,0662 g/l; CE = 20,86 g/l |
| Vorlage: | 574,8 g Avicel PH 102 |
| Sprührate: | 40-50 g/min (ungefährer Mittelwert) |
| relative Abluftfeuchte: | 70-80 % |
| Ablufttemperatur: | 32-34 °C |

Alle 3 Versuche verliefen unproblematisch. Die Sprühzeiten lagen bei Versuch 1 bei 83 Minuten, bei Versuch 2 bei 46 Minuten und bei Versuch 3 bei 35 Minuten.

### Versuch 4:

| | |
|---|---|
| eingesetzter Extrakt: | 4023,1 g; TS = 9,2 Gew.-%; |
| | Dichte: 1,0365 g/l; CE = 12,14 g/l |
| Vorlage: | 677,0 g Avicel PH 102 |
| Sprührate: | 40-50 g/min (ungefährer Mittelwert) |
| relative Abluftfeuchte: | 50-60 % |
| Ablufttemperatur: | 35-40 °C |

Dieser Versuch ist eine Wiederholung von Versuch 1, durch den Überprüft werden sollte, ob durch Verringerung der Sprührate ein feineres Trockenextrakt hergestellt werden kann. Der Trockenextrakt erwies sich in Siebanalysen gegenüber dem in Versuch 1 erhaltenen Trockenextrakt als feiner (siehe Zusammenfassung der Ergebnisse der Versuche).

Weitere Versuche wurden in analoger Verfahrensweise zu den Versuchen 1 bis 3 mit Avicel PH 102 (Versuch 5) oder mit Gemischen von Avicel PH 102 und Capsulac 60 (Gewichtsverhältnis 7:3; Versuch 6) durchgeführt.

### VERSUCHSERGEBNISSE

Detailergebnisse zur Hormonbilanz in den Versuchen 1 bis 4 sind in den Tabellen I bis IV zusammengefaßt.

Prinzipiell wurde gefunden, daß bei einer Vorlage von 570 g bis 680 g Avicel PH 102 als Trägerstoff ein kontinuierlicher und schneller Auftrag des Extraktes möglich ist (Versuche 1 bis 3). Für die eingesetzten von 1900 bis 4023 g variierten Extraktmengen lagen die Sprühzeiten für diese Versuche zwischen 35 und 83 Minuten. Hieraus ergaben sich Auftragsmengen von 0,55 g bis 0,64 g Feststoff aus dem Extrakt pro g Avicel (Mittelwert: 0,59 g).

Um in einem weiteren Versuch (Versuch 5) den vorgegebenen Soll-Gehalt von 45 mg konjugierte Oestrogene pro g Trockenextrakt einzuhalten bzw. Grenzen für maximal auftragbare Wirkstoffmengen zu ermitteln, wurde in.diesem Versuch die Vorlage an Avicel PH 102 auf 342,5 g reduziert - gegenüber den vorherigen Versuchen 1 bis 4 also eine Reduzierung um fast 50%. Aufgetragen werden sollte 4640 g Extrakt. Hierbei traten bis ca. 1600 g aufgesprühtem Extrakt keine Probleme auf, da bis zu dieser Menge wie in den vorangegangenen Versuchen 1 bis 4 wieder eine Auftragsmenge von 0,56 g Feststoff aus dem Extrakt pro g Avicel vorlag. Bei ca. 2000 g aufgesprühtem Extrakt ergab sich eine Auftragsmenge von 0,68 g und bei ca. 2500 g von 0,86 g Feststoff aus dem Extrakt pro g Avicel. Bis zu dieser Auftragsmenge ließ sich der Extrakt weitgehend unproblematisch aufsprühen. Danach wurde die Sprührate stark reduziert, da ab dieser Menge der Feststoff aus dem Extrakt die Menge des Trägerstoffes übersteigt und das Produkt ab diesem Punkt Klebeneigung zeigte. Der Prozeß wurde dann nur noch bei einer relativen Feuchte von < 25 % gefahren, da sich die Abluftfilter zusetzten; die Luftmenge war nicht mehr ausreichend um das Wirbelbett aufrecht zu erhalten. Die reine Sprühzeit betrug mehr als 5 Stunden.

Zusammenfassend kann daher gesagt werden, daß bis zu einem Auftrag von 0,6 g Feststoff aus dem Extrakt pro g Avicel der gesamte Extrakt verarbeitet sein sollte. Bei etwa 0,86 g Auftrag an Feststoff aus dem Extrakt liegt die Mengen-Obergrenze, die Avicel PH 102 an Extrakt ohne Beeinträchtigung aufnehmen kann. Danach ist eine Reduktion der Sprühauftrages und eine entsprechende Anpassung der restlichen Parameter erforderlich.

Versuch 4 stellt eine Wiederholung von Versuch 1 dar. Hier wurde durch Veränderung der Parameter (niedrigere Sprührate und dadurch höhere Ablufttemperatur und eine geringere Abluftfeuchte) eine feinere Verreibung hergestellt.

Beim zusätzlichen Versuch 6 erfolgte wie in Versuch 4 eine Reduktion der Vorlage, um auf 45 mg konjugierte Estrogene pro g Verreibung einstellen zu können (Reduktion > 60% gegenüber Versuch 1 und Versuch 2). Zusätzlich wurde hier Lactose eingesetzt (Verhältnis Avicel zu Lactose = 7 zu 3). In diesem Versuch war ab einer Auftragsmenge von 0,6 g Feststoff aus dem Extrakt pro g Gemisch Avicel/Lactose eine Absenkung der Sprührate von 20 g/min auf < 9 g/min erforderlich (bei V-140 lag die Grenze bei 0,86 g Feststoff). Die aufgesprühte Extraktmenge betrug zu diesem Zeitpunkt ca. 40 Gew.-% (< 1600 g). Ab ca. 1800 g trat auch hier, wie bereits in Versuch 4 beobachtet, Klebeneigung auf. Der Versuch wurde nach Auftrag von 70 % Extraktmenge abgebrochen, da eine weitere Absenkung der Sprührate (< 9 g/min) auf Grund der Geräte nicht möglich war.

**Tabelle I**

| Hormonbilanz für den Versuch 1 | | | | | | | | |
|---|---|---|---|---|---|---|---|---|
| **Oestrogene** | **Gesamtestrogene** | | | | **Freie Estrogene** | | | |
| | Lösungsextrakt | | Trockenextrakt | | Lösungsextrakt | | Trockenextrakt | |
| | [mg/g] | [%]¹⁾ | [mg/g] | [%]¹⁾ | [mg/g] | [%]²⁾ | [mg/g] | [%]²⁾ |
| 17-α-Estradiol | 0,455 | 4,34 | 1,856 | 4,31 | 0,047 | | 0,196 | |
| 17-β-Estradiol | 0,646 | 6,17 | 2,631 | 6,12 | 0,082 | | 0,344 | |
| 17-α-DH-Equilin | 1,270 | 12,12 | 5,160 | 11,99 | 0,098 | 0,94 | 0,405 | 0,94 |
| 17-β-DH-Equilin | 0,322 | 3,07 | 1,323 | 3,08 | 0,019 | | 0,077 | |
| 17-α-DH-Equilenin | 0,057 | 0,54 | 0,229 | 0,53 | 0,007 | | 0,021 | 0,53 |
| 17-β-DH-Equilenin | 0,031 | 0,30 | 0,215 | 0,50 | 0,000 | | 0,000 | |
| Estron | 6,193 | 59,12 | 25,371 | 58,97 | 0,247 | 2,36 | 1,015 | 2,36 |
| Equilin | 2,236 | 21,34 | 9,312 | 21,64 | 0,057 | 0,54 | 0,229 | |
| δ-8,9-Dehydroestron | 0,293 | 2,80 | 1,223 | 2,84 | 0,022 | | 0,076 | |
| Equilenin | 0,124 | 1,18 | 0,515 | 1,20 | 0,000 | | 0,000 | |
| Gesamthormongehalt | 11,627 | | 47,835 | | 0,579 | | 2,363 | |
| Summe Haupthormone³⁾ | 10,476 | | 43,022 | | 0,468 | | 1,922 | |

| | | | | | | | | |
|---|---|---|---|---|---|---|---|---|
| 1) bezogen auf 17-α-Estradiol, 17-α-DH-Equilin, 17-β-DH-Equilin, Estron und Equilin 2) bezogen auf Summe 17-α-Estradiol, 17-α-DH-Equilin, 17-β-DH-Equilin, Estron und Equilin aus Gesamtestrogenen 3) Summe der Hormone 17-α-Estradiol, 17-α-DH-Equilin, 17-β-DH-Equilin, Estron, Equilin | | | | | | | | |

**Tabelle II**

| Hormonbilanz für den Versuch 2 | | | | | | | | |
|---|---|---|---|---|---|---|---|---|
| **Oestrogene** | **Gesamtestrogene** | | | | **Freie Estrogene** | | | |
| | Lösungsextrakt | | Trockenextrakt | | Lösungsextrakt | | Trockenextrakt | |
| | [mg/g] | [%]¹⁾ | [mg/g] | [%]¹⁾ | [mg/g] | [%]²⁾ | [mg/g] | [%]²⁾ |
| 17-α-Estradiol | 0,826 | 4,46 | 2,085 | 4,49 | 0,087 | | 0,223 | 0,94 |
| 17-β-Estradiol | 1,220 | 6,58 | 3,074 | 6,62 | 0,163 | | 0,417 | |
| 17-α-DH-Equilin | 2,302 | 12,42 | 5,824 | 12,54 | 0,177 | 0,96 | 0,437 | |
| 17-β-DH-Equilin | 0,634 | 3,42 | 1,506 | 3,24 | 0,036 | | 0,087 | |
| 17-α-DH-Equilenin | 0,124 | 0,57 | 0,298 | 0,64 | 0,012 | | 0,030 | |
| 17-β-DH-Equilenin | 0,103 | 0,56 | 0,242 | 0,52 | 0,000 | | 0,000 | |
| Estron | 10,835 | 58,47 | 27,056 | 58,28 | 0,423 | 2,28 | 1,056 | 2,27 |
| Equilin | 3,934 | 21,23 | 9,957 | 21,45 | 0,092 | 0,50 | 0,223 | |
| δ-8,9-Dehydroestron | 0,529 | 2,85 | 1,348 | 2,90 | 0,016 | | 0,079 | 0,50 |
| Equilenin | 0,220 | 1,19 | 0,543 | 1,17 | 0,000 | | 0,000 | |
| Gesamthormongehalt | 20,727 | | 51,933 | | 1,006 | | 2,562 | |
| Summe Haupthormone³⁾ | 18,531 | | 46,428 | | 0,815 | | 2,036 | |

| | | | | | | | | |
|---|---|---|---|---|---|---|---|---|
| 1) bezogen auf 17-α-Estradiol,17-α-DH-Equilin, 17-β-DH-Equilin, Estron und Equilin 2) bezogen auf Summe 17-α-Estradiol, 17-α-DH-Equilin, 17-β-DH-Equilin, Estron und Equilin aus Gesamtestrogenen 3) Summe der Hormone 17-α-Estradiol, 17-α-DH-Equilin, 17-β-DH-Equilin, Estron, Equilin | | | | | | | | |

**Tabelle III**

| Hormonbilanz für den Versuch 3 | | | | | | | | |
|---|---|---|---|---|---|---|---|---|
| **Oestrogene** | **Gesamtestrogene** | | | | **Freie Estrogene** | | | |
| | Lösungsextrakt | | Trockenextrakt | | Lösungsextrakt | | Trockenextrakt | |
| | [mg/g] | [%]¹⁾ | [mg/g] | [%]¹⁾ | [mg/g] | [%]²⁾ | [mg/g] | [%]²⁾ |
| 17-α-Estradiol | 1,003 | 4,64 | 2,208 | 4,57 | 0,102 | | 0,227 | |
| 17-β-Estradiol | 1,402 | 6,49 | 3,072 | 6,36 | 0,166 | | 0,365 | |
| 17-α-DH-Equilin | 2,678 | 12,40 | 5,984 | 12,39 | 0,207 | 0,96 | 0,463 | 0,96 |
| 17-β-DH-Equilin | 0,633 | 2,93 | 1,432 | 2,96 | 0,038 | | 0,091 | |
| 17-α-DH-Equilenin | 0,118 | 0,55 | 0,232 | 0,48 | 0,021 | | 0,031 | |
| 17-β-DH-Equilenin | 0,045 | 0,21 | 0,057 | 0,12 | 0,000 | | 0,000 | |
| Estron | 12,713 | 58,87 | 28,105 | 58,18 | 0,492 | 2,28 | 1,083 | 2,24 |
| Equilin | 4,569 | 21,16 | 10,582 | 21,90 | 0,107 | 0,50 | 0,241 | 0,50 |
| δ-8,9-Dehydroestron | 0,539 | 2,50 | 1,265 | 2,62 | 0,021 | | 0,113 | |
| Equilenin | 0,222 | 1,03 | 0,492 | 1,02 | 0,000 | | 0,000 | |
| Gesamthormongehalt | 23,922 | | 53,429 | | 1.154 | | 2,614 | |
| Summe Haupthormone³⁾ | 21,596 | | 48,311 | | 0,946 | | 2,105 | |

| | | | | | | | | |
|---|---|---|---|---|---|---|---|---|
| 1) bezogen auf 17-α-Estradiol, 17-α-DH-Equilin, 17-β-DH-Equilin, Estron und Equilin 2) bezogen auf Summe 17-α-Estradiol, 17-α-DH-Equilin, 17-β-DH-Equilin, Estron und Equilin aus Gesamtestrogenen 3) Summe der Hormone 17-α-Estradiol, 17-α-DH-Equilin, 17-β-DH-Equilin, Estron, Equilin | | | | | | | | |

**Tabelle IV**

| Hormonbilanz für den Versuch 4 | | | | | | | | |
|---|---|---|---|---|---|---|---|---|
| **Oestrogene** | **Gesamtestrogene** | | | | **Freie Estrogene** | | | |
| | Lösungsextrakt | | Trockenextrakt | | Lösungsextrakt | | Trockenextrakt | |
| | [mg/g] | [%]¹⁾ | [mg/g] ¹⁾ | [%] | [mg/g] | [%] ²⁾ | [mg/g] | [%]²⁾ |
| 17-α-Estradiol | 0,646 | 4,56 | 2,284 | 4,56 | 0,057 | | 0,201 | |
| 17-β-Estradiol | 1,093 | 7,71 | 3,798 | 7,58 | 0,151 | | 0,531 | |
| 17-α-DH-Equilin | 1,876 | 13,23 | 6,367 | 12,71 | 0,134 | 0,94 | 0,484 | 0,97 |
| 17-β-DH-Equilin | 0,523 | 3,69 | 1,768 | 3,53 | 0,018 | | 0,109 | |
| 17-α-DH-Equilenin | 0,070 | 0,49 | 0,274 | 0,55 | 0,008 | | 0,030 | |
| 17-β-DH-Equilenin | 0,000 | 0,00 | 0,103 | 0,21 | 0,000 | | 0,000 | |
| Estron | 8,022 | 56,57 | 28,947 | 57,77 | 0,282 | 1,99 | 1,038 | 2,07 |
| Equilin | 3,114 | 21,96 | 10,743 | 21,44 | 0,068 | 0,50 | 0,229 | 0,46 |
| δ-8,9-Dehydroestron | 0,381 | 2,69 | 1,339 | 2,67 | 0,022 | | 0,083 | |
| Equilenin | 0,119 | 0,84 | 0,543 | 1,08 | 0,000 | | 0,000 | |
| Gesamthormongehalt | 15,844 | | 56,166 | | 0,740 | | 2,705 | |
| Summe Haupthormone³⁾ | 14,181 | | 50,109 | | | | 2,057 | |

| | | | | | | | | |
|---|---|---|---|---|---|---|---|---|
| 1) bezogen auf 17-α-Estradiol, 17-α-DH-Equilin, 17-β-DH-Equilin, Estron und Equilin 2) bezogen auf Summe 17-α-Estradiol, 17-α-DH-Equilin, 17-β-DH-Equilin, Estron und Equilin aus Gesamtestrogenen 3) Summe der Hormone 17-α-Estradiol, 17-α-DH-Equilin, 17-β-DH-Equilin, Estron, Equilin | | | | | | | | |

### BEWERTUNG DER VERSUCHSERGEBNISSE

Die Herstellung eines Trockenextraktes im Wirbelschichtgerät, auch mit Trägerstoffen unterschiedlicher Korngrößenverteilung, ist unproblematisch. Die Ausbeutebestimmung lag bei allen Versuchen zwischen 90 und 95%. Die Hormonverteilung, bezogen auf 17-α-DH-Equilin, Estron und Equilin, ist im Extrakt und in der Verreibung gleichbleibend. Der Trocknungsprozeß hat also keinen Einfluß auf die Stabilität der Hormone. Der Restfeuchtegehalt lag zwischen 3 und 6 % r.h..

Wie die Versuche zeigen, ist es möglich große Hormon-haltige Extraktmengen von 2 bis 4 kg innerhalb kurzer Zeit zu verarbeiten, d.h. auf Trägerstoffe aufzutragen und entsprechend zu trocknen. Als besonders wichtig hervorzuheben sind die ermittelten Auftragsmengen (g Feststoff aus dem Extrakt pro g Trägerstoff, z.B. Avicel), die maximal ohne Verfahrensprobleme auf den gewählten Trägerstoff aufgebracht werden können. Hier wurde festgestellt, daß bei Vorlage von z.B. Avicel als Trägerstoff ein Auftrag bis etwa 0,6 g Wirkstoff-Trockensubstanz aus dem hormon-haltigen wäßrigen Lösungsextrakt pro g Avicel völlig problemlos ist (Versuche 1, 2 und 3 des Beispiels 1).

### Beispiel 2:

### Versuche zur Tablettierung

Um die galenische Weiterverarbeitbarkeit der im Beispiel 1 durch Wirbelschichttechnologie hergestellten Trockenextrakte bzw. Präformulierungen zu testen, wurden die Trockenextrakte bzw. Präformulierungen mit weiteren Tablettierungshilfsstoffen vermischt und zu Matrixtabletten verpreßt. Es zeigte sich, daß die Mischungen in eine Matrixtablette homogen verteilt und verpreßt werden konnten. Überraschenderweise zeigte sich hierbei, daß durch die Wahl des Trägerstoffes und der weiteren Tablettierhilfsstoffe in Abhängigkeit der Wasserlöslichkeit des Träger- und Tablettierhilfsstoff-Gemisches die Freisetzungsgeschwindigkeit der in den Matrixtabletten verpreßten konjugierten Hormone entscheidend beeinflußt werden kann, und daß so erwünschte, vorgegebene Freisetzungsprofile eingestellt werden können. Auch die Zusammensetzung des für die konjugierten Oestrogene als Träger verwendeten Trägerstoffes, z.B. des Gemisches von mikrokristalliner Cellulose mit Lactose, die Partikelgröße und die Porosität des Wirkstoffgranulates, als auch die Partikelgrößenverteilung beeinflussen hierbei die Qualität der Verpreßbarkeit und das Freisetzungsprofil der Hormone, die aus der Matrix freigesetzt werden.

### Beispiel 3:

### Matrix-Tabletten mit kontrollierter Wirkstoff-Freisetzung

Die bevorzugte Präformulierung für die Herstellung von Matrix-Tabletten besteht aus einer in Beispiel 1 beschriebenen Trockenextraktfraktion abgemischt mit mikrokristalliner Cellulose als Wirkstoff, welcher mit den Hilfsstoffen mikrokristalliner Cellulose, Milchzucker (Lactose), Hydroxypropylcellulose Typ M fein in einem Wirbelschichtgranulator mit einer Hydroxypropylmethylcellulose-Lösung granuliert wird. Das hergestellte Granulat wird dann ggf. mit NaCl und Mg-Stearat gemischt und anschließend tablettiert. Die Tablette wird anschließend gewünschtenfalls mit einem Überzug in einen geeigneten Coater versehen, welcher aus HPMC (Hydroxypropylmethylcellulose), Polymethacrylatderivaten (z.B. Eudragit RL 30D), Polyethylenglykole wie z.B. 6000, Trithylcitrat und Talkum besteht.

Der Sinn der Befilmung der Matrix-Tabletten ist die Erzielung einer Verzögerung der Freisetzung (Steuerung der Blutspiegel im Körper) am Anfang des Freisetzungsverlaufes in Form einer erwünschten, vorgegebenen lag-time. Die Filmtablette wird zusätzlich abschließend zuckerdragiert, um eine ausreichende Stabilität der Hormon-Wirkung zu garantieren.

Es wurden in-vitro Freisetzungsuntersuchungen nach der U.S.P.-Methode in demineralisiertem Wasser durchgeführt. Ziel ist eine Steuerung der Freisetzungsgeschwindigkeit in Wasser, um die U.S.P.-Anforderungen zu erfüllen und ein vorgegebenes Freisetzungsprofil einzustellen. Da im Körper kein demineralisiertes Wasser, sondern osmotisch aktive Flüssigkeiten und ebenfalls unterschiedliche pH-Werte in Magen und Darm vorherrschen, wurden weitergehende Freisetzungen in osmotisch aktiven Medien und ggf. auch in Puffer-Medien durchgeführt.

Es zeigte sich, daß bei einer NaCl-haltigen Filmtablette der Unterschied der Freisetzung in demineralisiertem Wasser zu der Freisetzung in 0,9 %-NaCl-Lösung relativ gering ist.

Durch die Zugabe von NaCl in den äußeren Bereich der Matrix-Tablette, also durch Mischen mit dem hergestellten Granulat und Mg-Stearat, wird der Zug von Wasser in die Tablette nach Durchdringung des Films erhöht. Das schafft vor allem bei osmotisch aktiven Medien eine Gegenkraft und erzeugt einen erhöhten Quellungsdruck, der wiederum einen Druck auf den Film erzeugt. Der Film platzt somit nach Erzielung einer gewünschten lag-time durch den entstehenden Druck schneller auf und läßt so ein durch die genuine Tablette bedingtes Freisetzungsprofil zu.

### I. Matrix-Filmtabletten

### A. Filmtablette 0,625 mg (mit NaCl)

| Matrixkern | Menge in mg/Einzeldosis |
|---|---|
| Trockenextraktfraktion | 7,1 |
| Vivapur 101 | 7,5 |
| Vivapur 101 | 16,1 |
| Granulac 200 | 47,2 |
| HPC-M, fein | 31,2 |
| Pharmacoat Typ 603 | 3,4 |
| NaCl | 7,0 |
| MG-Stearat | 0,5 |
| | 120,0 |
| | 120,0 |

| Befilmung | Menge in mg/Einzeldosis |
|---|---|
| PEG 6000 | 0,28 |
| Methocel E5 | 0,948 |
| Triethylcitrat | 0,56 |
| Talkum, mikronisiert | 0,812 |
| Eudragit RL 30D | 0,9 |
| Wasser | -- |
| | 123,5 |

### B. Filmtablette 0,625 mg (ohne NaCl)

| Matrixkern | Menge in mg/Einzeldosis |
|---|---|
| Trockenextraktfraktion | 7,1 |
| Vivapur 101 | 7,5 |
| Vivapur 101 | 17,8 |
| Granulac 200 | 52,3 |
| HPC-M, fein | 31,2 |
| Pharmacoat.Typ 603 | 3,6 |
| MG-Stearat | 0,5 |
| | 120,0 |

| Befilmung | Menge in mg/Einzeldosis |
|---|---|
| PEG 6000 | 0,28 |
| Methocel E5 | 0,948 |
| Triethylcitrat | 0,56 |
| Talkum, mikronisiert | 0,812 |
| Eudragit RL 30D | 0,9 |
| Wasser | -- |
| | 123,5 |

### C. Filmtablette 0,625 mg (mit NaCl, Befilmung 1,5 mg/ED)

| Matrixkern | Menge in mg/Einzeldosis |
|---|---|
| Trockenextraktfraktion | 7,1 |
| Vivapur 101 | 7,5 |
| Vivapur 101 | 16,1 |
| Granulac 200 | 47,2 |
| HPC-M, fein | 31,2 |
| Pharmacoat Typ 603 | 3,4 |
| NaCl | 7,0 |
| MG-Stearat | 0,5 |
| | 120,0 |

| Befilmung | Menge in mg/Einzeldosis |
|---|---|
| Eudragit RL 30D | 0,615 |
| Triethylcitrat | 0,300 |
| PEG 6000 | 0,150 |
| Talkum, mikronisiert | 0,435 |
| | -- |
| Wasser | 121,5 |

### D. Matrixtablette 0,625 mg (ohne NaCl)

| Matrixkern | Menge in mg/Einzeldosis |
|---|---|
| Trockenextraktfraktion | 7,1 |
| Vivapur 101 | 7,5 |
| Vivapur 101 | 17,8 |
| Granulac 200 | 52,3 |
| HPC-M, fein | 31,2 |
| Pharmacoat Typ 603 | 3,6 |
| MG-Stearat | 0,5 |
| | 120,0 |

| Befilmung | Menge in mg/Einzeldosis | | |
|---|---|---|---|
| keine | | | |

### E. Matrixtablette 0,625 mg (mit NaCl, ohne Befilmung)

| Matrixkern | Menge in mg/Einzeldosis |
|---|---|
| Trockenextraktfraktion | 7,1 |
| Vivapur 101 | 7,5 |
| Vivapur 101 | 16,1 |
| Granulac 200 | 47,2 |
| HPC-M, fein | 31,2 |
| Pharmacoat Typ 603 | 3,4 |
| NaCl | 7,0 |
| MG-Stearat | 0,5 |
| | 120,0 |

| Befilmung | Menge in mg/Einzeldosis | | |
|---|---|---|---|
| keine | | | |

### F. Matrixtablette 0,625 mg (mit NaCl, ohne Befilmung)

| Matrixkern | Menge in mg/Einzeldosis |
|---|---|
| Trockenextraktfraktion | 7,1 |
| Vivapur 101 | 7,5 |
| Vivapur 101 | 16,0 |
| Granulac 200 | 47,1 |
| HPC-M, fein | 31, 2 |
| Pharmacoat Typ 603 | 3,6 |
| NaCl | 7,0 |
| MG-Stearat | 0,5 |
| | 120,0 |

| Befilmung | Menge in mg/Einzeldosis | | |
|---|---|---|---|
| keine | | | |

Tablette mit NaCl hergestellt, welches direkt beim Granulieren als Vorlage in der Mischung vorgegeben wurde.

### II. Wirkstofffreisetzungen

### Dissolutions-Test in 0,9 %iger NaCl Lösung

| | | | |
|---|---|---|---|
| | Testmethode: | Blattrührer | Drehgeschw.: 50 U/min |
| | Testmedium: | 0,9 % NaCl | Testvolumen: 900 ml |
| | Probevolumen | 10 ml | |
| | Tester: | Sotax AT 7 smart D 10 | |
| | | | |
| HPLC: | HPLC-Gerät: | H_LC_04 | |
| | Säulentyp: | LUNA 3µm C 18(2) | |

**Freisetzung von Estron: Matrix-Filmtablette A (mit NaCl)**

| Gefäß-Nr. | 1 | 2 | 3 | 4 | 5 | 6 |
|---|---|---|---|---|---|---|
| Gew.[mg] | 122.72 | 126.29 | 124.49 | 124.97 | 124.90 | 125.23 |
| 1 | 9.352 | 8.230 | 7.397 | 9.606 | 9.321 | 9.124 |
| 2 | 27.423 | 24.591 | 22.671 | 28.323 | 24.337 | 25.889 |
| 5 | 76.696 | 63.356 | 59.298 | 75.712 | 69.859 | 63.550 |
| 8 | 100.805 | 87.565 | 80.090 | 96.837 | 89.642 | 86.569 |

**Freisetzung von Estron: Matrix-Filmtablette B (ohne NaCl)**

| Gefäß-Nr. | 1 | 2 | 3 | 4 | 5 | 6 |
|---|---|---|---|---|---|---|
| Gew.[mg] | 125.14 | 124.03 | 126.15 | 124.83 | 126.95 | 127.34 |
| 1 | 7.957 | 8.391 | 7.602 | 7.197 | 9.997 | 7.883 |
| 2 | 21.605 | 23.843 | 20.862 | 20.437 | 20.2457 | 22.347 |
| 5 | 56.943 | 66.647 | 53.262 | 54.357 | 62.641 | 57.929 |
| 8 | 80.416 | 88.703 | 76.113 | 77.485 | 86.548 | 78.929 |

**Freisetzung von Estron: Matrix-Filmtablette C (mit NaCl, Coating ohne HPMC)**

| Gefäß-Nr. | 1 | 2 | 3 | 4 | 5 | 6 |
|---|---|---|---|---|---|---|
| Gew.[mg] | 120.14 | 122.29 | 121.92 | 121.76 | 120.60 | 121.64 |
| 1 | 5.233 | 3.808 | 3.437 | 3.977 | 3.783 | 4.528 |
| 2 | 15.569 | 12.260 | 10.913 | 12.757 | 12.708 | 16.240 |
| 5 | 72.993 | 67.216 | 62.390 | 72.676 | 71.998 | 77.769 |
| 8 | 88.783 | 86.697 | 83.630 | 90.331 | 89.873 | 90.329 |

**Freisetzung von Estron: Matrix-Filmtablette D (ohne NaCl, ohne Coating)**

| Gefäß-Nr. | 1 | 2 | 3 | 4 | 5 | 6 |
|---|---|---|---|---|---|---|
| Gew.[mg] | 119.63 | 120.09 | 120.55 | 120.48 | 119.71 | 121.21 |
| 1 | 34.299 | 35.517 | 35.024 | 36.913 | 34.113 | 34.618 |
| 2 | 52.663 | 53.096 | 52.718 | 56.150 | 52.136 | 52.285 |
| 5 | 89.068 | 88.165 | 86.086 | 92.987 | 87.094 | 88.125 |
| 8 | 101.905 | 100.326 | 99.713 | 103.604 | 97.611 | 102.261 |

**Freisetzung von Estron: Matrix-Filmtablette E (mit NaCl, ohne Coating)**

| Gefäß-Nr. | 1 | 2 | 3 | 4 | 5 | 6 |
|---|---|---|---|---|---|---|
| Gew.[mg] | 120.22 | 120.08 | 120.82 | 119.12 | 119.71 | 120.47 |
| 1 | 25.507 | 23.697 | 25.039 | 24.647 | 24.782 | 24.781 |
| 2 | 45.241 | 42.410 | 44.036 | 44.166 | 44.581 | 44.697 |
| 5 | 89.396 | 86.501 | 88.947 | 89.724 | 89.869 | 91.462 |
| 8 | 98.565 | 101.113 | 101.846 | 101.910 | 102.163 | 102.875 |

**Freisetzung von Estron: Matrix-Filmtablette F (mit NaCl, ohne Coating)**

| Gefäß-Nr. | 1 | 2 | 3 | 4 | 5 | 6 |
|---|---|---|---|---|---|---|
| Gew.[mg] | 120.16 | 120.51 | 120.65 | 119.25 | 121.07 | 120.84 |
| 1 | 26.502 | 50.097 | 24.346 | 25.389 | 21.941 | 25.461 |
| 2 | 48.852 | 69.102 | 42.665 | 44.772 | 39.979 | 44.818 |
| 5 | 95.432 | 94.347 | 87.589 | 89.842 | 80.394 | 87.613 |
| 8 | 102.050 | 94.248 | 101.189 | 99.147 | 89.638 | 95.833 |

## Patentansprüche

1. Pharmazeutische Matrix-Filmtablette mit kontrollierter Wirkstofffreisetzung, **dadurch gekennzeichnet, dass** die Tablette
(a) einen wasserquellbaren Matrixkern aufweist,
(i) der als Wirkstoff ein natürliches Gemisch konjugierter equiner Oestrogene aus dem Harn trächtiger Stuten in Form eines pharmazeutisch präformulierten homogenen Trockenextraktes eingebettet enthält, wobei der Trockenextrakt einen Wirkstoffgehalt des Gemisches natürlicher konjugierter equiner Oestrogene enthält,
welcher Wirkstoffgehalt pro Menge eines pharmazeutischen Trägerstoffes definiert ist, und bezogen auf die Haupthormon-Komponenten umfassend Estron, Equilin, 17-α-Dihydroequilin, und gegebenenfalls 17-α-Estradiol und 17-β-Dihydroequilin, jeweils in ihren freien und konjugierten Formen, standardisiert ist, und
welcher Wirkstoffgehalt durch Aufsprühen aus einer das Gemisch natürlicher konjugierter equiner Oestrogene enthaltenden wässrigen Lösung auf den pulver- und/oder granulatförmigen pharmazeutischen Trägerstoff aus der Gruppe mikrokristalliner Cellulosen oder einem Gemisch von mikrokristalliner Cellulose mit Lactose und Trocknung aufgebracht ist;
(ii) dessen wasserquellbare Matrix aus einer tablettierbaren Zusammensetzung gebildet wird, die wenigstens einen Gerüstbildner (Matrixbildner) aus der Gruppe gelbildender pharmazeutischer polymerer Trägerstoffe, sowie gegebenenfalls einen oder mehrere weitere pharmazeutische Tablettierhilfsstoffe aus der Gruppe anderer Gerüstbildner, der Füllstoffe, der Bindemittel, der wasserlöslichen osmotischen Agenzien und der Gleitmittel umfasst,
wobei der Matrixkern eine tablettierte Mischung des pharmazeutisch präformulierten homogenen Trockenextraktes mit wenigstens einem gelbildenden pharmazeutischen polymeren Trägerstoff als Gerüstbildner (Matrixbildner) und gegebenenfalls einem oder mehreren weiteren pharmazeutischen Tablettierhilfsstoffen darstellt,
und dass die Tablette
(b) mit einem den Matrixkern umgebenden, film-bildenden Überzug versehen ist, dessen Zusammensetzung
(i) wenigstens einen hydrophoben pharmazeutische Filmbildner und weiterhin
(ii) gegebenenfalls pharmazeutische Weichmacher und/oder Porenbildner und/oder ein hydrophiles Polymer
umfasst.

2. Matrix-Filmtablette nach Anspruch 1, **dadurch gekennzeichnet, dass** der gel-bildende pharmazeutische polymere Trägerstoff ausgewählt ist aus der Gruppe der Cellulosederivate und/oder Stärkederivate, vorzugsweise aus der Gruppe der Cellulosederivate, und insbesondere aus der Gruppe umfassend Hydroxypropylcellulose (HPC), Hydroxypropylmethylcellulose (HPMC) und Carboxymethylcellulose (CMC).

3. Matrix-Filmtablette nach Anspruch 1 oder 2, **dadurch gekennzeichnet, dass** die wasserquellbare Matrix als weitere pharmazeutische Tablettierhilfsstoffe eine oder mehrere mikrokristalline Cellulosen umfasst.

4. Matrix-Filmtablette nach Anspruch 1, **dadurch gekennzeichnet, dass** die wasserquellbare Matrix als weitere pharmazeutische Tablettierhilfsstoffe Gleitmittel aus der Gruppe der Fließregulierungsmittel, Schmiermittel, und/oder Formtrennmittel umfasst.

5. Matrix-Filmtablette nach einem der vorhergehenden Ansprüche, **dadurch gekennzeichnet, dass** der Matrixkern wenigstens einen gel-bildenden pharmazeutischen polymeren Trägerstoff aus der Gruppe der Cellulosederivate und als weitere pharmazeutische Tablettierhilfsstoffe wenigstens eine mikrokristalline Cellulose als zusätzlichen Trägerstoff umfasst.

6. Matrix-Filmtablette nach einem der vorhergehenden Ansprüche, **dadurch gekennzeichnet, dass** der Matrixkern wenigstens einen gel-bildenden pharmazeutischen polymeren Trägerstoff aus der Gruppe der Cellulosederivate und als weitere pharmazeutische Tablettierhilfsstoffe Lactose als wasserlösliches Agens umfasst.

7. Matrix-Filmtablette nach einem der vorhergehenden Ansprüche, **dadurch gekennzeichnet, dass** der Matrixkern wenigstens einen gel-bildenden pharmazeutischen polymeren Trägerstoff aus der Gruppe der Cellulosederivate und als weitere pharmazeutische Tablettierhilfsstoffe Natriumchlorid als osmotisches Agens umfasst.

8. Matrix-Filmtablette nach einem der vorhergehenden Ansprüche, **dadurch gekennzeichnet, dass** der Matrixkern wenigstens einen gel-bildenden pharmazeutischen polymeren Trägerstoff aus der Gruppe der Cellulosederivate und als weitere pharmazeutische Tablettierhilfsstoffe wenigstens eine mikrokristalline Cellulose als zusätzlichen Trägerstoff, Lactose als wasserlösliches Agens und gegebenenfalls Natriumchlorid als osmotisches Agens umfasst.

9. Matrix-Filmtablette nach einem der vorhergehenden Ansprüche 1 bis 5, **dadurch gekennzeichnet, dass** die wasserquellbare Matrix 20 bis 50 Gew.-Teile eines gel-bildenden pharmazeutischen polymeren Trägerstoffes aus der Gruppe der Cellulosederivate und als weitere pharmazeutische Tablettierhilfsstoffe 10 bis 30 Gew.-Teile einer mikrokristallinen Cellulose und 40 bis 70 Gew.-Teile eines wasserlöslichen Agens und gegebenenfalls 0,1 bis 3 Gew.-Teile eines osmotischen Agens enthält.

10. Matrix-Filmtablette nach Anspruch 9, **dadurch gekennzeichnet, daß** die wasserquellbare Matrix 20 bis 50 Gew.-Teile Hydroxypropylcellulose (HPC) als gelbildenden pharmazeutischen polymeren Trägerstoff und als weitere pharmazeutische Hilfsstoffe 10 bis 30 Gew.-Teile mikrokristalline Cellulose und 40 bis 70 Gew.-Teile Lactose als wasserlösliches Agens und 0,1 bis 3 Gew.-Teile Natriumchlorid als osmotisches Agens enthält.

11. Matrix-Filmtablette nach einem der vorhergehenden Ansprüche, **dadurch gekennzeichnet, dass** der Matrixkern als weiteren pharmazeutische Tablettierhilfsstoff ein Schmiermittel, vorzugsweise Magnesiumstearat, enthält.

12. Matrix-Filmtablette nach Anspruch 11, **dadurch gekennzeichnet, dass** das Schmiermittel in einer Menge im Matrixkern enthalten ist, die bezogen auf 100 Gew.-Teile der wasserquellbaren Matrix 0,1 bis 5 Gew.-Teile, vorzugsweise 2 bis 5 Gew.-Teile entspricht.

13. Matrix-Filmtablette nach Anspruch 1, **dadurch gekennzeichnet, dass** der Überzug als hydrophoben pharmazeutischen Filmbildner ein Polymethacrylat, als Weichmacher Triethylcitrat und einen Porenbildner aus der Gruppe Polyethylenglykol 6000 (PEG 6000) und/oder Hydroxypropylmethylcellulose umfasst.

14. Matrix-Filmtablette nach Anspruch 13, **dadurch gekennzeichnet, dass** der Überzug weiterhin Talkum enthält.

15. Matrix-Filmtablette nach Anspruch 13, **dadurch gekennzeichnet, daß** der Überzug bezogen auf den Matrixkern als 100 Gew.-% das Polymethacrylat in einer Menge von 0,1 bis 1 Gew.-%, das Triethylcitrat in einer Menge von 0,05 bis 0,5 Gew.-% und Polyethylenglykol 6000 in einer Menge von 0,01 bis 0,5 Gew.-% und/oder Hydroxypropylmethylcellulose in einer Menge von 0,01 bis 0,5 Gew.-% enthält.

16. Matrix-Filmtablette nach Anspruch 1, **dadurch gekennzeichnet, daß** die Matrix-Filmtablette einschließlich Überzug in einer Tablettenstärke mit einem Gesamtgewicht an Wirkstoffgehalt von 0,3 mg, 0,625 mg, 0,9 mg, 1,25 mg oder 2,5 mg des natürlichen Gemisches konjugierter equiner Östrogene pro Tablette vorliegt.

17. Matrix-Filmtablette nach Anspruch 16, **dadurch gekennzeichnet, daß** die Matrix-Filmtablette für die Tablettenstärken von 0,3 mg und 0,625 mg ein Freisetzungsprofil mit einer als Summe von Estron und Equilin gemessenen Wirkstofffreisetzung von 19 bis 49 % in 2 Stunden, 66 bis 96 % in 5 Stunden und > 80 % nach 8 Stunden aufweist.

18. Matrix-Filmtablette nach Anspruch 16, **dadurch gekennzeichnet, daß** die Matrix-Filmtablette für die Tablettenstärken von 0,9 mg und 0,625 mg ein Freisetzungsprofil mit einer als Summe von Estron und Equilin gemessenen Wirkstofffreisetzung von 12 bis 37 % in 2 Stunden, 57 bis 85 % in 5 Stunden und > 80 % nach 8 Stunden aufweist.

19. Matrix-Filmtablette nach Anspruch 16. **dadurch gekennzeichnet, daß** die Matrix-Filmtablette für die Tablettenstärken von 1,25 mg und 2,5 mg ein Freisetzungsprofil mit einer als Summe von Estron und Equilin gemessenen Wirkstofffreisetzung von 3 bis 22 % in 2 Stunden, 37 bis 67 % in 5 Stunden, 6 bis 96 % in 8 Stunden und > 80 % nach 12 Stunden aufweist.

20. Matrix-Filmtablette nach einem der Ansprüche 1 bis 19, **dadurch gekennzeichet, daß** die Matrix-Filmtablette zusätzlich eine Zuckerdragierung aufweist.

21. Verfahren zur Herstellung einer Matrix-Filmtablette gemäß einem der Ansprüche 2 bis 19, **dadurch gekennzeichnet, daß** man
(a) als Wirkstoff ein natürliches Gemisch konjugierter equiner Oestrogene aus dem Harn trächtiger Stuten, das in Form eines pharmazeutisch präformulierten homogenen Trockenextraktes vorliegt, wobei der Trockenextrakt einen Wirkstoffgehalt des Gemisches natürlicher konjugierter equiner Oestrogene enthält,
welcher Wirkstoffgehalt pro Menge eines pharmazeutischen Trägerstoffes definiert ist, und bezogen auf die Haupthormon-Komponenten umfassend Estron, Equilin, 17-α-Dihydroequilin, und gegebenenfalls 17-α-Estradiol und 17-β-Dihydroequilin, jeweils in ihren freien und konjugierten Formen, standardisiert ist, und
welcher Wirkstoffgehalt durch Aufsprühen aus einer das Gemisch natürlicher konjugierter equiner Oestrogene enthaltenden wäßrigen Lösung auf einen pulver- und/oder granulatförmigen pharmazeutischen Trägerstoff aus der Gruppe mikrokristalliner Cellulosen oder einem Gemisch von mikrokristalliner Cellulose mit Lactose und Trocknung aufgebracht wurde,
und als Gerüstbildner (Matrixbildner) wenigstens einen gel-bildenden pharmazeutischen polymeren Trägerstoff aus der Gruppe der Cellulose- und/oder Stärkederivate sowie gegebenenfalls einen oder mehrere weitere pharmazeutische Tablettierhilfsstoffe aus der Gruppe zusätzlicher Trägerstoffe in Form von mikrokristallinen Cellulosen, wasserlöslicher osmotischer Agenzien und gegebenenfalls Bindemittel und/oder Schmiermittel gleichzeitig oder in beliebiger Reihenfolge miteinander vermischt, gewünschtenfalls auch unter Zusatz einer Polymerlösung, vorzugsweise einer Hydroxypropylmethylcellulose-Lösung oder eines Hydroxypropylmethylcellulose Sols,
(b) die unter (a) erhaltene, gegebenenfalls bereits granulierte Mischung nachfolgend in einer Tablettiermaschine zu Matrixkernen verpreßt, und
(c) die unter (b) erhaltenen Matrixkerne mit einem Überzug aus einer Filmzusammensetzung beschichtet, die wenigstens einen hydrophoben pharmazeutischen Filmbildner und weiterhin gegebenenfalls einen pharmazeutischen Weichmacher und/oder Porenbildner umfaßt.

22. Verfahren nach Anspruch 21, **dadurch gekennzeichnet, daß** man auf die nach Anspruch 21 hergestellten Matrix-Filmtabletten zusätzlich eine Zuckerdragierung aufbringt.

## Claims

1. Pharmaceutical matrix film-coated tablet with controlled active ingredient release, **characterized in that** the tablet
(a) has a water-swellable matrix core,
(i) which comprises as active ingredient a natural mixture of conjugated equine oestrogens from pregnant mares' urine incorporated in the form of a pharmaceutically preformulated homogeneous dry extract, where the dry extract comprises an active ingredient content of the mixture of natural conjugated equine oestrogens, which active ingredient content is defined per quantity of a pharmaceutical carrier and is standardized in relation to the principal hormone components including oestrone, equilin, 17-α-dihydroequilin, and optionally 17-α-estradiol and 17-β-dihydroequilin, in each case in the free and conjugated forms thereof, and
which active ingredient content is applied, by spraying from an aqueous solution comprising the mixture of natural conjugated equine oestrogens, to the pharmaceutical carrier which is in powder and/or granule form and is from the group of microcrystalline celluloses or a mixture of microcrystalline cellulose with lactose and drying;
(ii) the water-swellable matrix thereof is formed from a tablettable composition which includes at least one matrix former from the group of gel-forming pharmaceutical polymeric carriers, and optionally one or more further pharmaceutical tabletting aids from the group of other matrix formers, of fillers, of binders, of water-soluble osmotic agents and of glidants,
where the matrix core represents a tabletted mixture of the pharmaceutically preformulated homogeneous dry extract with at least one gel-forming pharmaceutical polymeric carrier as matrix former and optionally one or more further pharmaceutical tabletting aids,
and **in that** the tablet
(b) is provided with a film-forming coating which surrounds the matrix core and whose composition includes
(i) at least one hydrophobic pharmaceutical film former and furthermore
(ii) optionally pharmaceutical plasticizers and/or pore formers and/or a hydrophilic polymer.

2. Matrix film-coated tablet according to Claim 1, **characterized in that** the gel-forming pharmaceutical polymeric carrier is selected from the group of cellulose derivatives and/or starch derivatives, preferably from the group of cellulose derivatives, and in particular from the group including hydroxypropylcellulose (HPC), hydroxypropylmethylcellulose (HPMC) and carboxymethylcellulose (CMC).

3. Matrix film-coated tablet according to Claim 1 or 2, **characterized in that** the water-swellable matrix includes as further pharmaceutical tabletting aids one or more microcrystalline celluloses.

4. Matrix film-coated tablet according to Claim 1, **characterized in that** the water-swellable matrix includes as further pharmaceutical tabletting aids glidants from the group of flow regulators, lubricants, and/or mould release agents.

5. Matrix film-coated tablet according to any of the preceding claims, **characterized in that** the matrix core includes at least one gel-forming pharmaceutical polymeric carrier from the group of cellulose derivatives and as further pharmaceutical tabletting aids at least one microcrystalline cellulose as additional carrier.

6. Matrix film-coated tablet according to any of the preceding claims, **characterized in that** the matrix core includes at least one gel-forming pharmaceutical polymeric carrier from the group of cellulose derivatives and as further pharmaceutical tabletting aids lactose as water-soluble agent.

7. Matrix film-coated tablet according to any of the preceding claims, **characterized in that** the matrix core includes at least one gel-forming pharmaceutical polymeric carrier from the group of cellulose derivatives and as further pharmaceutical tabletting aids sodium chloride as osmotic agent.

8. Matrix film-coated tablet according to any of the preceding claims, **characterized in that** the matrix core includes at least one gel-forming pharmaceutical polymeric carrier from the group of cellulose derivatives and as further pharmaceutical tabletting aids at least one microcrystalline cellulose as additional carrier, lactose as water-soluble agent and optionally sodium chloride as osmotic agent.

9. Matrix film-coated tablet according to any of preceding Claims 1 to 5, **characterized in that** the water-swellable matrix comprises from 20 to 50 parts by weight of a gel-forming pharmaceutical polymeric carrier from the group of cellulose derivatives and as further pharmaceutical tabletting aids from 10 to 30 parts by weight of a microcrystalline cellulose and from 40 to 70 parts by weight of a water-soluble agent and optionally from 0.1 to 3 parts by weight of an osmotic agent.

10. Matrix film-coated tablet according to Claim 9, **characterized in that** the water-swellable matrix comprises from 20 to 50 parts by weight of hydroxypropylcellulose (HPC) as gel-forming pharmaceutical polymeric carrier and as further pharmaceutical aids from 10 to 30 parts by weight of microcrystalline cellulose and from 40 to 70 parts by weight of lactose as water-soluble agent and from 0.1 to 3 parts by weight of sodium chloride as osmotic agent.

11. Matrix film-coated tablet according to any of the preceding claims, **characterized in that** the matrix core comprises as further pharmaceutical tabletting aid a lubricant, preferably magnesium stearate.

12. Matrix film-coated tablet according to Claim 11, **characterized in that** the quantity of lubricant contained in the matrix core corresponds to from 0.1 to 5 parts by weight, preferably 2 to 5 parts by weight, based on 100 parts by weight of the water-swellable matrix.

13. Matrix film-coated tablet according to Claim 1, **characterized in that** the coating includes as hydrophobic pharmaceutical film former a polymethacrylate, as plasticizer triethyl citrate and a pore former from the group of polyethylene glycol 6000 (PEG 6000) and/or hydroxypropylmethylcellulose.

14. Matrix film-coated tablet according to Claim 13, **characterized in that** the coating further comprises talc.

15. Matrix film-coated tablet according to Claim 13, **characterized in that** the coating comprises, based on the matrix core as 100% by weight, the polymethacrylate in a quantity of from 0.1 to 1% by weight, the triethyl citrate in a quantity of from 0.05 to 0.5% by weight and polyethylene glycol 6000 in a quantity of from 0.01 to 0.5% by weight and/or hydroxypropylmethylcellulose in a quantity of from 0.01 to 0.5% by weight.

16. Matrix film-coated tablet according to Claim 1, **characterized in that** the matrix film-coated tablet including coating is present in a tablet strength with a total weight of active ingredient content of 0.3 mg, 0.625 mg, 0.9 mg, 1.25 mg or 2.5 mg of the natural mixture of conjugated equine oestrogens per tablet.

17. Matrix film-coated tablet according to Claim 16, **characterized in that** the matrix film-coated tablet has for the tablet strengths of 0.3 mg and 0.625 mg a release profile with an active ingredient release, measured as the total of oestrone and equilin, of from 19 to 49% in 2 hours, 66 to 96% in 5 hours and > 80% after 8 hours.

18. Matrix film-coated tablet according to Claim 16, **characterized in that** the matrix film-coated tablet has for the tablet strengths of 0.9 mg and 0.625 mg a release profile with an active ingredient release, measured as the total of oestrone and equilin, of from 12 to 37% in 2 hours, 57 to 85% in 5 hours and > 80% after 8 hours.

19. Matrix film-coated tablet according to Claim 16, **characterized in that** the matrix film-coated tablet has for the tablet strengths of 1.25 mg and 2.5 mg a release profile with an active ingredient release, measured as the total of oestrone and equilin, of from 3 to 22% in 2 hours, 37 to 67% in 5 hours, 6 to 96% in 8 hours and > 80% after 12 hours.

20. Matrix film-coated tablet according to any of Claims 1 to 19, **characterized in that** the matrix film-coated tablet additionally has a sugar coating.

21. Process for producing a matrix film-coated tablet according to any of Claims 2 to 19, **characterized in that**
(a) as active ingredient a natural mixture of conjugated equine oestrogens from pregnant mares' urine which is present in the form of a pharmaceutically preformulated homogeneous dry extract, where the dry extract comprises an active ingredient content of the mixture of natural conjugated equine oestrogens,
which active ingredient content is defined per quantity of a pharmaceutical carrier and is standardized in relation to the principal hormone components including oestrone, equilin, 17-α-dihydroequilin, and optionally 17-α-estradiol and 17-β-dihydroequilin, in each case in the free and conjugated forms thereof, and
which active ingredient content has been applied, by spraying from an aqueous solution comprising the mixture of natural conjugated equine oestrogens, to a pharmaceutical carrier which is in powder and/or granule form and is from the group of microcrystalline celluloses or a mixture of microcrystalline cellulose with lactose and drying,
and as matrix former at least one gel-forming pharmaceutical polymeric carrier from the group of cellulose derivatives and/or starch derivatives, and optionally one or more further pharmaceutical tabletting aids from the group of additional carriers in the form of microcrystalline celluloses, water-soluble osmotic agents and optionally binders and/or lubricants are mixed together simultaneously or in any sequence,
if desired also with the addition of a polymer solution, preferably of a hydroxypropylmethylcellulose solution or of a hydroxypropylmethylcellulose sol,
(b) the mixture obtained under (a), which is optionally already granulated, is subsequently compressed to matrix cores in a tabletting machine, and
(c) the matrix cores obtained under (b) are coated with a coating of a film composition which includes at least one hydrophobic pharmaceutical film former and furthermore optionally a pharmaceutical plasticizer and/or pore former.

22. Process according to Claim 21, **characterized in that** a sugar coating is additionally applied to the matrix film-coated tablets produced according to Claim 21.

## Revendications

1. Comprimé pelliculé pharmaceutique à matrice avec une libération contrôlée de la substance active, **caractérisé en ce que** le comprimé
(a) présente un noyau de matrice gonflable à l'eau,
(i) qui contient sous forme enrobée, comme substance active, un mélange naturel d'estrogènes équins conjugués provenant de l'urine de juments pleines sous forme d'un extrait sec homogène pharmaceutiquement préformulé, l'extrait sec contenant une teneur en substance active du mélange d'estrogènes équins conjugués naturels,
la teneur en substance active étant définie par quantité d'une substance support pharmaceutique et étant standardisée par rapport aux composants hormonaux principaux, comprenant l'estron, l'équiline, la 17-α-dihydroéquiline, et le cas échéant le 17-α-estradiol et la 17-β-dihydroéquiline, à chaque fois dans leurs formes libres et conjuguées, et
la teneur en substance active étant appliquée par pulvérisation d'une solution aqueuse contenant le mélange d'estrogènes équins conjugués naturels sur la substance support pharmaceutique sous forme de poudre et/ou de granulés du groupe des celluloses microcristallines ou un mélange de cellulose microcristalline avec du lactose et par séchage ;
(ii) dont la matrice gonflable à l'eau est formée par une composition compressible, qui contient au moins un agent de formation de la structure (agent de formation de la matrice) du groupe des substances support polymères pharmaceutiques gélifiantes, ainsi que le cas échéant un ou plusieurs autres adjuvants de compression pharmaceutiques du groupe des autres agents de formation de la structure, des charges, des liants, des agents osmotiques solubles dans l'eau et des agents de glisse,
le noyau de matrice représentant un mélange comprimé de l'extrait sec homogène pharmaceutiquement préformulé avec au moins une substance support polymère pharmaceutique gélifiante comme agent de formation de la structure (agent de formation de la matrice) et le cas échéant un ou plusieurs adjuvants de compression pharmaceutiques,
et **en ce que** le comprimé
(b) est pourvu d'un revêtement filmogène entourant le noyau de matrice, dont la composition comprend
(i) au moins un agent filmogène pharmaceutique hydrophobe et en outre
(ii) le cas échéant des plastifiants et/ou des agents de formation de pores et/ou un polymère hydrophile pharmaceutique(s).

2. Comprimé pelliculé à matrice selon la revendication 1, **caractérisé en ce que** la substance support polymère pharmaceutique gélifiante est choisie dans le groupe des dérivés de cellulose et/ou des dérivés d'amidon, de préférence dans le groupe des dérivés de cellulose et en particulier dans le groupe comprenant l'hydroxypropylcellulose (HPC), l'hydroxypropylméthylcellulose (HPMC) et la carboxyméthylcellulose (CMC).

3. Comprimé pelliculé à matrice selon la revendication 1 ou 2, **caractérisé en ce que** la matrice gonflable à l'eau comprend comme autres adjuvants de compression pharmaceutiques une ou plusieurs celluloses microcristallines.

4. Comprimé pelliculé à matrice selon la revendication 1, **caractérisé en ce que** la matrice gonflable à l'eau comprend comme autres adjuvants de compression pharmaceutiques des agents de glisse du groupe des agents de régulation de l'écoulement, des lubrifiants et/ou des agents de démoulage.

5. Comprimé pelliculé à matrice selon l'une quelconque des revendications précédentes, **caractérisé en ce que** le noyau de la matrice comprend au moins une substance support polymère pharmaceutique gélifiante du groupe des dérivés de cellulose et comme autres adjuvants de compression pharmaceutiques au moins une cellulose microcristalline comme substance support supplémentaire.

6. Comprimé pelliculé à matrice selon l'une quelconque des revendications précédentes, **caractérisé en ce que** le noyau de la matrice comprend au moins une substance support polymère pharmaceutique gélifiante du groupe des dérivés de cellulose et comme autres adjuvants de compression pharmaceutiques du lactose comme agent soluble dans l'eau.

7. Comprimé pelliculé à matrice selon l'une quelconque des revendications précédentes, **caractérisé en ce que** le noyau de la matrice comprend au moins une substance support polymère pharmaceutique gélifiante du groupe des dérivés de cellulose et comme autres adjuvants de compression pharmaceutiques du chlorure de sodium comme agent osmotique.

8. Comprimé pelliculé à matrice selon l'une quelconque des revendications précédentes, **caractérisé en ce que** le noyau de la matrice comprend au moins une substance support polymère pharmaceutique gélifiante du groupe des dérivés de cellulose et comme autres adjuvants de compression pharmaceutiques au moins une cellulose microcristalline comme substance support supplémentaire, du lactose comme agent soluble dans l'eau et le cas échéant du chlorure de sodium comme agent osmotique.

9. Comprimé pelliculé à matrice selon l'une quelconque des revendications précédentes 1 à 5, **caractérisé en ce que** la matrice gonflable à l'eau contient 20 à 50 parties en poids d'une substance support polymère pharmaceutique gélifiante du groupe des dérivés de cellulose et comme autres adjuvants de compression pharmaceutiques 10 à 30 parties en poids d'une cellulose microcristalline et 40 à 70 parties en poids d'un agent soluble dans l'eau et le cas échéant 0,1 à 3 parties en poids d'un agent osmotique.

10. Comprimé pelliculé à matrice selon la revendication 9, **caractérisé en ce que** la matrice gonflable à l'eau contient 20 à 50 parties en poids d'hydroxypropylcellulose (HPC) comme substance support polymère pharmaceutique gélifiante et comme autres adjuvants pharmaceutiques 10 à 30 parties en poids de cellulose microcristalline et 40 à 70 parties en poids de lactose comme agent soluble dans l'eau et 0,1 à 3 parties en poids de chlorure de sodium comme agent osmotique.

11. Comprimé pelliculé à matrice selon l'une quelconque des revendications précédentes, **caractérisé en ce que** le noyau de la matrice contient comme autre adjuvant de compression pharmaceutique un lubrifiant, de préférence le stéarate de magnésium.

12. Comprimé pelliculé à matrice selon la revendication 11, **caractérisé en ce que** le lubrifiant est contenu en une quantité dans le noyau de la matrice qui, par rapport à 100 parties en poids de la matrice gonflable à l'eau, correspond à 0,1 jusqu'à 5 parties en poids, de préférence à 2 jusqu'à 5 parties en poids.

13. Comprimé pelliculé à matrice selon la revendication 1, **caractérisé en ce que** le revêtement comprend, comme agent filmogène pharmaceutique hydrophobe un polyméthacrylate, comme plastifiant du citrate de triéthyle et un agent de formation de pores du groupe formé par le polyéthylèneglycol 6000 (PEG 6000) et/ou l'hydroxypropylméthylcellulose.

14. Comprimé pelliculé à matrice selon la revendication 13, **caractérisé en ce que** le revêtement contient en outre du talc.

15. Comprimé pelliculé à matrice selon la revendication 13, **caractérisé en ce que** le revêtement contient, par rapport au noyau de la matrice comme 100% en poids, le polyméthacrylate en une quantité de 0,1 à 1% en poids, le citrate de triéthyle en une quantité de 0,05 à 0,5% en poids et le polyéthylèneglycol 6000 en une quantité de 0,01 à 0,5% en poids et/ou l'hydroxypropylméthylcellulose en une quantité de 0,01 à 0,5% en poids.

16. Comprimé pelliculé à matrice selon la revendication 1, **caractérisé en ce que** le comprimé pelliculé à matrice y compris le revêtement se trouve dans un calibre de comprimé avec un poids total de teneur en substance active de 0,3 mg, 0,625 mg, 0,9 mg, 1,25 mg ou 2,5 mg du mélange naturel d'estrogènes équins conjugués par comprimé.

17. Comprimé pelliculé à matrice selon la revendication 16, **caractérisé en ce que** le comprimé pelliculé à matrice, pour les calibres de comprimé de 0,3 mg et 0,625 mg, présente un profil de libération avec une libération de substance active mesurée comme la somme d'estron et d'équiline de 19 à 49% en 2 heures, de 66 à 96% en 5 heures et > 80% après 8 heures.

18. Comprimé pelliculé à matrice selon la revendication 16, **caractérisé en ce que** le comprimé pelliculé à matrice, pour les calibres de comprimé de 0,9 mg et 0,625 mg, présente un profil de libération avec une libération de substance active mesurée comme la somme d'estron et d'équiline de 12 à 37% en 2 heures, de 57 à 85% en 5 heures et > 80% après 8 heures.

19. Comprimé pelliculé à matrice selon la revendication 16, **caractérisé en ce que** le comprimé pelliculé à matrice, pour les calibres de comprimé de 1,25 mg et 2,5 mg, présente un profil de libération avec une libération de substance active mesurée comme la somme d'estron et d'équiline de 3 à 22% en 2 heures, de 37 à 67% en 5 heures, de 6 à 96% en 8 heures et > 80% après 12 heures.

20. Comprimé pelliculé à matrice selon l'une quelconque des revendications 1 à 19, **caractérisé en ce que** le comprimé pelliculé à matrice présente en outre un enrobage en sucre.

21. Procédé pour la préparation d'un comprimé pelliculé à matrice selon l'une quelconque des revendications 2 à 19, **caractérisé en ce qu'**on
(a) mélange les uns avec les autres comme substance active un mélange naturel d'estrogènes équins conjugués provenant de l'urine de juments pleines, qui se trouve sous forme d'un extrait sec homogène pharmaceutiquement préformulé, l'extrait sec contenant une teneur en substance active du mélange d'estrogènes équins conjugués naturels,
la teneur en substance active étant définie par quantité d'une substance support pharmaceutique et étant standardisée par rapport aux composants hormonaux principaux, comprenant l'estron, l'équiline, la 17-α-dihydroéquiline, et le cas échéant le 17-α-estradiol et la 17-β-dihydroéquiline, à chaque fois dans leurs formes libres et conjuguées, et
la teneur en substance active étant appliquée par pulvérisation d'une solution aqueuse contenant le mélange d'estrogènes équins conjugués naturels sur une substance support pharmaceutique sous forme de poudre et/ou de granulés du groupe des celluloses microcristallines ou un mélange de cellulose microcristalline avec du lactose et par séchage ;
et comme agent de formation de la structure (agent de formation de la matrice) au moins une substance support polymère pharmaceutique gélifiante du groupe des dérivés de cellulose et/ou d'amidon
ainsi que le cas échéant un ou plusieurs autres adjuvants de compression pharmaceutiques du groupe des substances support supplémentaires sous forme de celluloses microcristallines, des agents osmotiques solubles dans l'eau et le cas échéant des liants et/ou des lubrifiants, simultanément ou dans un ordre quelconque
si souhaité également avec addition d'une solution polymère, de préférence une solution d'hydroxypropylméthylcellulose ou un sol d'hydroxypropylméthylcellulose,
(b) comprime ensuite le mélange obtenu au point (a), le cas échéant déjà granulé dans une machine de compression en noyaux de matrice, et
(c) recouvre le noyau de matrice obtenu au point b) par un revêtement d'une composition filmogène, qui comprend au moins un agent filmogène pharmaceutique hydrophobe et en outre le cas échéant un plastifiant pharmaceutique et/ou agent de formation de pores.

22. Procédé selon la revendication 21, **caractérisé en ce qu'**on applique en outre sur les comprimés pelliculés à matrice préparés selon la revendication 21 un enrobage en sucre.
